(19) **Europäisches Patentamt / European Patent Office / Office européen des brevets**

(11) **EP 4 353 837 A2**

(12) # EUROPEAN PATENT APPLICATION

(43) Date of publication:
**17.04.2024 Bulletin 2024/16**

(21) Application number: **24155625.7**

(22) Date of filing: **19.02.2018**

(51) International Patent Classification (IPC):
***C12Q 1/6883*** (2018.01)

(52) Cooperative Patent Classification (CPC):
(C-Sets available)
**C12Q 1/6883**  (Cont.)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.02.2017  US 201762460887 P**

(62) Document number(s) of the earlier application(s) in accordance with Art. 76 EPC:
**18711014.3 / 3 583 231**

(71) Applicant: **NEOVENTURES BIOTECHNOLOGY EUROPE**
**94800 Villejuif (FR)**

(72) Inventor: **PENNER, Gregory**
**London N6B 2T5 (CA)**

(74) Representative: **Icosa**
**83 avenue Denfert-Rochereau**
**75014 Paris (FR)**

Remarks:
•The complete document including Reference Table(s) and the Sequence Listing(s) can be downloaded from the EPO website
•This application was filed on 02-02-2024 as a divisional application to the application mentioned under INID code 62.

(54) **APTAMER AS BIOMARKERS**

(57)     The present invention relates to the use of the change in relative frequency of aptamers in a library selected against at least one sample from at least one reference subject of known outcome, prior to and following selection of said library against a sample from a subject, as a means of diagnosing a medical state, disease or condition in said subject.

The present invention also relates to methods for diagnosing a medical state, disease or condition in a subject, comprising correlating changes in relative frequency between aptamers to a medical state, disease or condition and diagnosing said subject based on the determined correlations.

**EP 4 353 837 A2**

(52) Cooperative Patent Classification (CPC): (Cont.)

C-Sets
**C12Q 1/6883, C12Q 2525/205, C12Q 2537/16**

**Description**

**FIELD OF INVENTION**

The present invention relates to the identification of aptamers that may be used as biomarkers to diagnose a pathology or to diagnose sub-types of a pathology, or to determine the rate of progression of a pathology within subjects.
More specifically, the present invention relates to methods for diagnosing a pathology, for diagnosing sub-types of a pathology, or for determining the rate of progression of a pathology within subjects, which methods comprise the use of a set of aptamers enriched in sequences that bind to epitopes in a bodily fluid or tissue and further enriched against samples of the same bodily fluid or tissue from subjects that vary for a phenotype; and the correlation of variation in the relative frequency of enriched aptamers' frequencies and variation in the phenotype across such subjects.

**BACKGROUND OF INVENTION**

Strimbu and Tavel (Curr Opin HIV AIDS. 2010 Nov; 5(6):463-466) define biomarkers as "*objective indications of medical state observed from outside the patient - which can be measured accurately and reproducibly*". This definition does not require that the functional relationship between the biomarker and the medical state be understood, the definition only requires that the relationship between the measurement of the biomarker and the medical state be measured accurately and reproducibly.
There are two primary types of biomarkers that are known in the art: one, termed "genomic analysis", is based on the correlation of variants in genomic sequences and medical states across patients. The second type of biomarker consists of a measurement of the abundance of a protein or metabolite, where such abundance has been correlated with a medical state. In the case of genomic analysis, the correlation is based on the prediction of a protein sequence. The physical basis for the correlation is assumed to be the effect of the sequence at the protein level on the function of the protein. For protein or metabolite biomarkers, the physical basis for the correlation is assumed to be an outcome of a change in expression level, or expression specificity, or a post-translational modification.
In either case, in general, the basis of the utility of the biomarker for medical diagnosis is a correlation between a measured biomarker and a medical state, not a physical cause and effect relationship. Biomarkers may also be used to assign a probability of a phenotype rather than a diagnosis *per se.* As an example, high cholesterol levels in the blood are used to predict the probability of cardiac failure, not as a diagnosis of cardiac failure.
In the present invention, the Inventors disclose a new type of biomarker that fulfils the same general basis of utility for medical diagnosis and/or for predicting the probability of a medical state.
The present invention is enabled by the use of aptamer libraries that have been enriched for their capacity to bind to epitopes in a bodily fluid or tissue. Aptamers are short oligomers usually formed from nucleic acids (DNA, RNA, PNA or a mix thereof) that exhibit the capacity to bind to a specific epitope. A key advantage for aptamers over antibodies for the co-discovery of ligands and biomarkers is that the identification of aptamers is based on the re-iterative selection of very large random pools of potential ligands. As such, it is possible to expose a random aptamer library to a tissue that exhibits a pathology and to select aptamers that bind to epitopes within this tissue. Moreover, it is possible to impose counter-selection on such a library by exposing it to tissue that does not exhibit the pathology (*i.e.,* a substantially healthy tissue) and selecting against sequences that bind to such a tissue. Alternating selection against binding to substantially healthy tissue, and selection for binding to tissue affected by a pathology results in the enrichment of sequences that bind to epitopes that are specifically enriched in the tissue affected by the pathology. As such, it is possible to develop selected aptamer libraries that bind to a broad range of epitopes in a tissue affected by a pathology. This same process of enriching aptamer sequences for pathological epitopes also applies to the selection of aptamers for such epitopes in bodily fluids from patients affected by a given pathology with counter-selection against the same bodily fluid from individuals unaffected by said given pathology. A method to enable the selection of aptamers in bodily fluids is provided in International patent application WO2017035666.
The term "epitope" has arisen from immunology and is generally taken to mean the site on a target molecule which is recognized and bound to by an antibody. This concept applies *mutatis mutandis* for aptamers.
The enrichment of individual aptamer sequences in response to selection against pathological epitopes is characterized by measuring the relative frequency of individual sequences in a sample from the library through a process such as next generation sequencing (NGS). The relative frequency of each aptamer within a selected library of aptamers is determined by dividing the number of times that a given aptamer sequence is observed (copy number) by the total number of aptamer sequences observed within a library. The enrichment of aptamers within libraries in successive selection rounds for pathological epitopes is a function of each aptamers ability to bind to a positive target, and the relative frequency or concentration of the target in the sample used for selection.
The insight that is the basis of the present invention is that differences in relative frequency of specific aptamers arising from simultaneous selection of an aptamer library against different patient samples must be due to differences in epitope frequency within the patients. Differences in binding affinity would result in the same change in relative frequency given equal epitope frequency across patients. That is, the selection process will lead to changes in the relative frequency of aptamers within a selection library as a result of differences in binding affinity of the aptamers and differences in epitope concentration across patients. Differences in the relative frequency of aptamers across patients in simultaneous selections in the same selection round can only be due to differences in the relative frequency of the epitopes that they bind to.

As such, it was our insight that given the characterization of aptamers that have differences in change in relative frequency between a selected library and specific patient libraries between substantially healthy individuals and individuals affected with a specific pathology, the change in relative frequency of such aptamers could be used to confer a diagnosis of a medical state, or to assign a probability of such a medical state occurring.

The Inventors have named aptamers that are characterized as useful for the diagnosis of a given pathology as "AptaMarkers".

The change in relative frequency of such AptaMarkers is a function of the selection process on specific patients on the aptamers present in the selected library. There is competition among aptamers for pathogenic epitopes. If a sample has a higher level of a pathological epitope than other samples, then aptamers that bind to this pathological epitope will be enriched to a higher level in such a sample than in other samples. Conversely, if a sample has a lower level of a pathological epitope than other samples, then aptamers that bind to this pathological epitope will be enriched to a lower level in such a sample than in other samples. Moreover, it follows that given that the present invention is based on the relative frequency of such aptamers in the set of sequences characterized by the sequencing process, that if certain aptamers are enriched to a higher level in a given sample because the epitope that they bind to is present at a higher concentration in said sample, that other aptamers that bind to epitopes whose concentration is the same across samples would be enriched less. This is to say that, if any given aptamer is enriched in relative frequency, there must be a compensatory decrease in the relative frequency of other aptamers.

The relative frequency of each aptamer in the library is then affected by the relative frequency of all the other aptamers in the library. As such, the present invention is based on changes in the relative frequencies of Aptamarkers within the context of a library, but not their relative change in and of themselves.

It is also our insight that the change in the relative frequency of AptaMarkers could be used to confer a diagnosis of the level of severity of a pathology. The level of change in the relative frequency of AptaMarkers is a function of the frequency of the pathogenic epitopes that they bind to. As such, the level of change in the relative frequency of an AptaMarker can be correlated with the pathogenic epitope concentration. In comparison with clinical data, algorithms can be developed, whereby the level of change in the relative frequency of a set of AptaMarkers is used to generate a score that is indicative of the level of severity of the pathology.

It is also our insight that the change in the relative frequency of AptaMarkers within individual patients over time could be used to develop a projection of the rate of the progression of a pathology. Specific patients may exhibit different rates of progression of a given pathology. By performing repeated analysis of such patients over time, the rate of change in the AptaMarkers can be determined. A patient exhibiting a higher rate of change in AptaMarkers can be predicted to be demonstrating a higher rate of pathology progression. As such, algorithms can be developed that will correlate the rate of change in AptaMarkers to the rate of change in a pathology.

It is also our insight that the change in relative frequency of AptaMarkers across individual patients could be used to stratify patients. Different patients may exhibit different pathological epitopes for the same pathology. As such, it is possible to use the change in relative frequency of AptaMarkers to characterize clusters of similar patients, and to corollary identify different clusters of patients within a pathology. This information would be of value for the testing of potential therapies for the pathology and a more targeted selection of subjects for participation in clinical researches. This application of Aptamarkers could be useful for the stratification of patients that could be correlated with their level of response to a treatment.

It is also our insight that the change in relative frequency of AptaMarkers across individual patients receiving a therapy could be used to evaluate the efficacy of such a therapy. A measurement of the change in relative frequency in an individual patient prior to such a patient receiving the therapy, and on a repeated basis over time once the patient is receiving the therapy, can be used to determine which AptaMarkers are affected by the therapy, how much the AptaMarkers are affected, whether there is a correlation between effect on AptaMarker and dosage of the therapy, or method of application of the therapy and the level of effectiveness of the therapy. This information on the change in relative frequency of AptaMarkers is informative because the AptaMarkers are binding to pathogenic epitopes.

As such, it would be possible to identify patients affected by a pathology that respond to a specific therapy and to use the AptaMarker response of such patients as a predictive indicator for the selection of patients for the application of the same therapy. This would be of value to the developers of therapies.

Others have attempted to develop methods for the use of aptamers to identify biomarkers but not for the use of aptamers as biomarkers in themselves.

Notably, U.S. patent application US20150087536 describes a method for the identification and quantification of proteins in bodily fluids through the use of aptamers. This method is limited however to the use of aptamers for which their binding to known proteins has already been established. Pools of such aptamers are combined with proteins derived from a bodily fluid and allowed to bind. Unbound aptamers are removed by washing. The remaining bound aptamers are sequenced and the identity and quantity of the proteins present in the bodily fluid are determined by the presence and quantity of the aptamers as revealed by sequencing.

The present invention represents an improvement over the invention disclosed in U.S. patent application US20150087536, in that the present invention first and foremost involves the selection of aptamers for uncharacterized pathogenic epitopes in tissues or bodily fluids. The Inventors have enabled the characterization of changes in the abundance of such pathological epitopes by measuring changes in the frequency of the aptamers that bind to them. US20150087536 is however limited in application to those aptamers for which known protein targets have been identified only. The present invention is not limited by the development of known aptamers that are known to bind to given proteins.

Moreover, US20150087536 is limited to aptamers that bind to purified proteins. The present invention is not limited in this manner. The Inventors have indeed been able to detect the abundance of proteins in complexes with other biological entities, such as other proteins or metabolites, or cell membranes. The present invention is also not limited to proteins, since the Inventors were able to detect portions of pathogenic epitopes that involve complexes among metabolites, metabolites alone, or metabolites in association with cellular membranes.

In addition, the present invention is able to detect the abundance of peptide fragments of proteins whose abundance is enriched by a pathology. As an example, in the case of neurological disorders, proteins are often cleaved into peptides for removal through the brain-blood-barrier. These peptides exist in the blood, and could be a target of the present invention as pathological epitopes. Aptamers selected against purified proteins often will not bind to peptides derived from such proteins, as the epitope presented by the peptide may differ from the epitope presented by the purified protein.

A publication by Berezovski et al., (J Am Chem Soc. 2008 Jul 16;130(28):9137-43) describes a method for the selection of enriched pools of aptamers, and the use of these enriched pools to identify biomarkers. A library of aptamers is selected against proteins in blood serum from patients with a pathology. This library is synthesized with a biotin group for subsequent immobilization on streptavidin-coated magnetic beads. The library is first incubated with proteins from bodily fluid and then the protein/aptamer complexes are removed from this incubation by the introduction of the magnetic beads. The bound proteins are identified by liquid chromatography/mass spectrometry (LC-MS) analysis.

The disclosure of Berezovski et al. differs from the present invention in that, while it uses aptamers to identify potential protein biomarkers, it does not directly develop a method for diagnosis or quantification of these biomarkers.

The method of Berezovski et al. is also limited to the identification of specific proteins, but not of peptides, metabolites, complexes between proteins and metabolites or proteins with other proteins.

The method of Berzovski et al. is also limited to the use of aptamers as a tool to discover proteins with the scope being limited to the subsequent use of such proteins as biomarkers.

The concept of using the aptamers that bind to these proteins as biomarkers was not contemplated by these authors.

The present invention is capable of detecting differential folding of a protein, and is therefore not limited to just the identification of a given protein. It is well-known in the art that the misfolding of certain proteins, or peptides derived from such proteins, may be indicative of a pathology, such as, e.g., amyloid beta peptides (Aβ) and pTau in the case of Alzheimer's disease. Both Aβ plaques and tau tangles provide examples of sources of pathological epitopes that are related to a folded state of a protein rather than the protein itself. The present invention has the capacity to directly measure the abundance of these pathological epitopes, while the method of Berzovski et al. does not.

Facing the limitations set forth in the prior art, there remains an unmet need for the development of a new method to more accurately diagnosing a medical state, assigning a probability of such a medical state occurring, diagnosing the level of severity of a pathology, developing a projection of the rate of the progression of a pathology, stratifying patients, evaluating the efficacy of a therapy and/or identifying patients affected by a pathology that respond to a specific therapy.

Here, the Inventors have developed a method that uses aptamers that bind to targets within bodily fluids or tissues as biomarkers in themselves. The use of aptamers as biomarkers is based on correlations between the relative frequency of an aptamer or set of aptamers within a library of aptamers following a single round of selection against a bodily fluid or tissue and a phenotype or medical state.

As such, the present invention has circumvented the need to know what the aptamer is binding to in the bodily fluid or tissue. Indeed, methods previously disclosed entail a means of using aptamers to identify a protein, which protein is then used as a biomarker. However, the present invention broadens the scope of potential targets to anything capable of presenting an epitope, and circumvents the need to identify what the target is.

Moreover, the identification of potential targets in bodily fluids and tissues can be difficult as a result of the low concentration of the target, and the difficulty associated with characterizing such a target in a complex mix of targets.

The present invention overcomes this difficulty by enabling characterization of variation in the target that is correlated with variation in a medical state without requiring identification of the target.

## SUMMARY

The present invention relates to the use of the change in relative frequency of aptamers in a library selected against at least one sample from at least one reference subject of known outcome, prior to and following selection of said library against a sample from a subject, as a means of diagnosing a medical state, disease or condition in said subject.
The present invention also relates to a method for diagnosing a medical state, disease or condition in a subject, comprising the steps of:

- providing a selected aptamer library, wherein the selected aptamer library comprises a collection of aptamer sequences selected and optionally counter-selected against a bodily tissue or fluid from at least one reference subject,
- contacting the selected aptamer library with a biological sample from at least one subject with known outcome for the medical state, disease or condition,
- selecting aptamers from the selected aptamer library that bind to the biological sample, thereby obtaining a subject-specific aptamer library,
- determining the change in relative frequency between aptamers from the selected aptamer library and the subject-specific aptamer library,
- correlating changes in relative frequency between aptamers from the selected aptamer library and the subject-specific aptamer library to the medical state, disease or condition of the at least one subject, and
- applying the selected aptamer library to subjects for which the medical state, disease or condition is unknown, thereby diagnosing the subject for the medical state, disease or condition based on the correlations determined in the previous step between the relative change of aptamers within the library and said medical state, disease or condition.

The present invention also relates to a method for diagnosing a medical state, disease or condition in a subject, comprising the steps of:

- providing a selected aptamer library, wherein the selected aptamer library comprises a collection of aptamer sequences selected and optionally counter-selected against a bodily tissue or fluid from at least one reference subject,
- contacting the selected aptamer library with a biological sample from at least two subjects with known outcome for the medical state, disease or condition,
- selecting aptamers from the selected aptamer library that bind to the biological sample, thereby obtaining at least two subject-specific aptamer libraries,
- determining the change in relative frequency between aptamers from the at least two subject-specific aptamer libraries,
- correlating changes in relative frequency between aptamers within subject-specific aptamer libraries to the medical state disease or condition of the at least two subjects, and
- applying the selected aptamer library to subjects for which the medical state, disease or condition is unknown, thereby diagnosing the subject for the medical state, disease or condition based on the correlations determined in the previous step between the relative change of aptamers within the library and said medical state, disease or condition.

In one embodiment, the at least one reference subject is a subject with known outcome for the medical state, disease or condition.
In one embodiment, the medical state, disease or condition is a neurodegenerative disease.
In one embodiment, the medical state, disease or condition is Alzheimer's disease.
In one embodiment, the step of contacting the selected aptamer library with a biological sample comprises two or more selection rounds.
In one embodiment, the selected aptamer library comprises a subset of known and characterized aptamer sequences at known ratios.
In one embodiment, the change in relative frequency is determined at various time points, thereby assessing the rate of progression of the medical state, disease or condition in the subject.
In one embodiment, the uses and methods of the present invention are for assigning a probability of the medical state, disease or condition to occur in the subject.
In one embodiment, the uses and methods of the present invention are for diagnosing the level of severity of the medical state, disease or condition in the subject.
In one embodiment, the uses and methods of the present invention are for determining the rate of progression of the medical state, disease or condition in the subject.

In one embodiment, the uses and methods of the present invention are for stratifying the subject affected with the medical state, disease or condition.

In one embodiment, the uses and methods of the present invention are for evaluating the efficacy of a therapy in the subject affected with the medical state, disease or condition.

**DEFINITIONS**

In the present invention, the following terms have the following meanings:
The term "**aptamer**", as used herein, refers to oligonucleotides that mimic antibodies in their ability to act as ligands and bind to analytes. In one embodiment, aptamers comprise natural DNA nucleotides, natural RNA nucleotides, modified DNA nucleotides, modified RNA nucleotides, or a combination thereof.

The terms "**selected library**" or "**selected aptamer library**", as used herein, refer to a collection of aptamer sequences that have been exposed to a target, where such a target may be a bodily tissue or bodily fluid, through a process known in the art as aptamer selection, and where such a selected library exhibits the characteristic that at least 0.01% of the sequences observed in a sample of one million sequences are observed again in a subsequent selection round against the same target.

The terms "**subject-specific aptamer library**", "**patient-specific aptamer library**" or "**diagnostic library**", as used herein, refer to a collection of aptamer sequences that represent an aliquot from a selected library that has been applied at least once in a process of aptamer selection to a target, where such a target may be a bodily tissue or fluid from an individual.

The term "**relative frequency**" as used herein, with respect to an aptamer, refers to the copy number of that aptamer in a sample of sequences from either a selected aptamer library or a subject-specific aptamer library divided by the total number of sequences observed.

The term "**change in relative frequency**" as used herein, with respect to an aptamer, refers to the relative frequency of that aptamer in a subject-specific aptamer library compared to the relative frequency of the same aptamer in either a selected aptamer library or another subject-specific aptamer library.

The term "**aptamer cluster**", as used herein, refers to a population or pool of aptamers with different sequences, where all the relative frequency of the aptamers within an "aptamer cluster" exhibit a statistically significant level of covariance across subject-specific aptamer libraries.

The term "**subject**" refers to a warm-blooded animal, preferably a human, a pet or livestock. As used herein, the terms "pet" and "livestock" include, but are not limited to, dogs, cats, guinea pigs, rabbits, pigs, cattle, sheep, goats, horses and poultry. In some embodiments, the subject is a male or female subject. In some embodiments, the subject is an adult (for example, a subject above the age of 18 (in human years) or a subject after reproductive capacity has been attained). In another embodiment, the subject is a child (for example, a subject below the age of 18 (in human years) or a subject before reproductive capacity has been attained). In one embodiment, the subject is above the age of 20, preferably above the age of 30, 40, 50, 60, 70, 80, 90 years old or more. In one embodiment, the subject is from 30 to 90 years old, preferably from 40 to 90 years old, more preferably from 50 to 90 years old, even more preferably from 60 to 90 years old, even more preferably from 70 to 90 years old. In some embodiments, the subject may be a "**patient**", *i.e.,* a subject who/which is awaiting the receipt of, or is receiving medical care or was/is/will be the object of a medical or diagnostic procedure according to the methods of the present invention, or is monitored for the development of a disease.

**DETAILED DESCRIPTION**

The present invention relates to the use of the change in relative frequency of aptamers in a library selected against at least one sample from at least one reference subject of known outcome, prior to and following selection of said library against a sample from a subject, as a means of diagnosing a medical state, disease or condition in said subject.

The present invention also relates to a method for diagnosing a medical state, disease or condition in a subject.

The present invention also relates to a method for assigning a probability of a medical state, disease or condition to occur in a subject.

The present invention also relates to a method for diagnosing the level of severity of a medical state, disease or condition in a subject.

The present invention also relates to a method for determining the rate of progression of a medical state, disease or condition in a subject.

The present invention also relates to a method for stratifying subjects affected with a medical state, disease or condition.

The present invention also relates to a method for evaluating the efficacy of a therapy in a subject affected with a medical state, disease or condition.

In one embodiment, the uses and methods of the present invention comprise the steps of:

- providing a selected aptamer library,
- contacting the selected aptamer library with a biological sample from at least one subject with known outcome for a medical state, disease or condition,
- selecting aptamers from the selected aptamer library that bind to the biological sample, thereby obtaining a subject-specific aptamer library, and
- determining the change in relative frequency between aptamers from the selected aptamer library and the subject-specific aptamer library,
- correlating changes in relative frequency between aptamers from the selected aptamer library and the subject-specific aptamer library to the medical state, disease or condition of the at least one subject, and
- applying the selected aptamer library to subjects for which the medical state, disease or condition is unknown, thereby providing a diagnosis based on the correlations determined in the previous step between the relative change of aptamers within the library and said medical state, disease or condition.

In one embodiment, the uses and methods of the present invention comprise the steps of:

- providing a selected aptamer library, wherein the selected aptamer library comprises a collection of aptamer sequences selected and optionally counter-selected against a bodily tissue or fluid from a reference subject,
- contacting the selected aptamer library with a biological sample from at least one subject with known outcome for a medical state, disease or condition,
- selecting aptamers from the selected aptamer library that bind to the biological sample, thereby obtaining a subject-specific aptamer library,
- determining the change in relative frequency between aptamers from the selected aptamer library and the subject-specific aptamer library,
- correlating changes in relative frequency between aptamers from the selected aptamer library and the subject-specific aptamer library to the medical state, disease or condition of the at least one subject, and
- applying the selected aptamer library to subjects for which the medical state, disease or condition is unknown, thereby providing a diagnosis based on the correlations determined in the previous step between the relative change of aptamers within the library and said medical state, disease or condition.

In one embodiment, the uses and methods of the present invention comprise the steps of:

- providing a selected aptamer library,
- contacting the selected aptamer library with a biological sample from at least two subjects with known outcome for a medical state, disease or condition,
- selecting aptamers from the selected aptamer library that bind to the biological sample, thereby obtaining at least two subject-specific aptamer libraries,
- determining the change in relative frequency between aptamers from the at least two subject-specific aptamer libraries,
- correlating changes in relative frequency between aptamers within subject-specific aptamer libraries to the medical state disease or condition of the at least two subjects, and
- applying the selected aptamer library to subjects for which the medical state, disease or condition is unknown, thereby providing a diagnosis based on the correlations determined in the previous step between the relative change of aptamers within the library and said medical state, disease or condition.

In one embodiment, the uses and methods of the present invention comprise the steps of:

- providing a selected aptamer library, wherein the selected aptamer library comprises a collection of aptamer sequences selected and optionally counter-selected against a bodily tissue or fluid from a reference subject,
- contacting the selected aptamer library with a biological sample from at least two subjects with known outcome for a medical state, disease or condition,
- selecting aptamers from the selected aptamer library that bind to the biological sample, thereby obtaining at least two subject-specific aptamer libraries,
- determining the change in relative frequency between aptamers from the at least two subject-specific aptamer libraries,
- correlating changes in relative frequency between aptamers within subject-specific aptamer libraries to the medical state disease or condition of the at least two subjects, and

- applying the selected aptamer library to subjects for which the medical state, disease or condition is unknown, thereby providing a diagnosis based on the correlations determined in the previous step between the relative change of aptamers within the library and said medical state, disease or condition.

In one embodiment, the uses and methods of the invention are implemented on samples from individuals (hereafter referred to as "**diagnostic subjects**") whose level of pathology is unknown, in order to characterize the following:

a) is the patient affected by the pathology?
b) if the answer to a) is yes, then what level of pathological effect is the patient exhibiting? and
c) if the answer to a) is yes, then samples from the patient shall be processed over regular time intervals to determine the rate of change in the relevant aptamer frequencies, This information can be used, *e.g.,* to project the individual rate of increase in degree of affectation by the pathology.

These steps provide a basis for creating a framework for the use of an aptamer set as biomarkers.
In one embodiment, the uses and methods of the invention comprise a step of providing a selected aptamer library. In one embodiment, the selected aptamer library is a commercially available selected aptamer library. In one embodiment, the selected aptamer library is prepared *ab initio.*
In one embodiment, the selected aptamer library was obtained or was prepared through a process of aptamer selection.
In one embodiment, the selected aptamer library comprises, after aptamer selection, at least 0.001%, at least 0.005%, at least 0.01%, at least 0.05%, at least 0.1%, at least 0.5%, at least 1% of the sequences comprised in a library of sequences. In one embodiment, the selected aptamer library comprises, after aptamer selection, at least 0.001%, at least 0.005%, at least 0.01%, at least 0.05%, at least 0.1%, at least 0.5%, at least 1% of the sequences comprised in a library of at least 100 000 sequences, at least 250 000 sequences, at least 500 000 sequences, at least 750 000 sequences, at least 1 000 000 sequences, at least 1 250 000 sequences, at least 1 500 000 sequences, at least 1 750 000 sequences, at least 2 000 000 sequences. In a preferred embodiment, the selected aptamer library comprises, after aptamer selection, at least 0.01% of the sequences comprised in a library of one million sequences.
As used herein, a "**selected aptamer library**" refers to a library of aptamer sequences which are specific for at least one epitope.
In one embodiment, the at least one epitope is a pathological epitope. The term "**pathological epitope**" refers to an epitope which is characteristic of the medical state, disease or condition under investigation.
It is contemplated in the present invention that various medical state, disease or condition can be investigated using any one of the uses and methods described herein. Therefore, the term "**medical state, disease or condition under investigation**" is used herein to refer to a given medical state, disease or condition for which a diagnosis is to be determined in a given subject.
In one embodiment, the selected aptamer library comprises aptamer sequences specific for at least one epitope, at least 2, at least 3, at least 4, at least 5, at least 10, at least 20, at least 30 or more epitopes, preferably pathological epitopes.
In one embodiment, the medical state, disease or condition include, but are not limited to, neurodegenerative diseases, neurological diseases, cancers, autoimmune diseases, cardiovascular diseases, infections, inflammatory diseases and metabolic diseases.
Examples of neurodegenerative diseases include, but are not limited to, Alzheimer's disease, mild cognitive impairment (MCI), impaired memory functions, Parkinson's disease, Huntington's disease, multiple sclerosis, amyotrophic lateral sclerosis (ALS) (including familial ALS and sporadic ALS), Pick's disease, dementia, depression, sleep disorders, psychoses, epilepsy, schizophrenia, paranoia, attention deficit hyperactivity disorder (ADHD), amnesiac syndromes, progressive supranuclear palsy, brain tumor, head trauma and Lyme disease.
In a preferred embodiment, the medical state, disease or condition is Alzheimer's disease.
In one embodiment, the selected aptamer library is prepared *ab initio.*
In one embodiment, the selected aptamer library is prepared by reiterative selection of a random set of oligonucleotide sequences against a bodily tissue or fluid from at least one reference subject. In one embodiment, the selected aptamer library is prepared by reiterative counter-selection of a random set of oligonucleotide sequences against a bodily tissue or fluid from at least one reference subject.
In one embodiment, the selected aptamer library is prepared by alternating reiterative selection and reiterative counter-selection of a random set of oligonucleotide sequences against a bodily tissue or fluid from at least one reference subject.
In one embodiment, the reiterative selection comprises at least one round, at least 2, 3, 4, 5, 6, 7, 8, 9, 10, 11, 12, 13, 14, 15, 16, 17, 18, 19, 20, 25, 30, 35, 40, 50 rounds or more of selection against a bodily tissue or fluid from at least one reference subject. In one embodiment, at least 2, 3, 4, 5, 6, 7, 8, 9, 10, 11, 12, 13, 14, 15, 16, 17, 18, 19, 20, 25, 30, 35, 40, 50 rounds or more of selection are carried out against a bodily tissue or fluid from different reference subjects. In one embodiment, each round of selection is carried out against a bodily tissue or fluid from another reference subject.

In one embodiment, the reiterative counter-selection comprises at least one round, at least 2, 3, 4, 5, 6, 7, 8, 9, 10, 11, 12, 13, 14, 15, 16, 17, 18, 19, 20, 25, 30, 35, 40, 50 rounds or more of counter-selection against a bodily tissue or fluid from at least one reference subject. In one embodiment, at least 2, 3, 4, 5, 6, 7, 8, 9, 10, 11, 12, 13, 14, 15, 16, 17, 18, 19, 20, 25, 30, 35, 40, 50 rounds or more of counter-selection are carried out against a bodily tissue or fluid from different reference subjects. In one embodiment, each round of counter-selection is carried out against a bodily tissue or fluid from another reference subject.

In one embodiment, the preparation of the selected aptamer library comprises at least one round, at least 2, 3, 4, 5, 6, 7, 8, 9, 10, 11, 12, 13, 14, 15, 16, 17, 18, 19, 20, 25, 30, 35, 40, 50 rounds or more of alternating reiterative selection and reiterative counter-selection against a bodily tissue or fluid from at least one reference subject. In one embodiment, at least 2, 3, 4, 5, 6, 7, 8, 9, 10, 11, 12, 13, 14, 15, 16, 17, 18, 19, 20, 25, 30, 35, 40, 50 rounds or more of alternating reiterative selection and reiterative counter-selection are carried out against a bodily tissue or fluid from different reference subjects. In one embodiment, each round of alternating reiterative selection and reiterative counter-selection is carried out against a bodily tissue or fluid from another reference subject.

Examples of bodily tissues include, but are not limited to, brain, heart, lungs, muscles, intestines, stomach, kidneys, skin, gonads, nerve cells, cornea, retina and other structures of the eye, tendons, bone, bone marrow, nail-base cells, cartilage, non-fetal maternity related tissues such as placenta, umbilical cord, foreskin, surfaces of internal lumens, vascular tissue, pancreas, spleen, adrenal gland, thyroid gland and pituitary glands. In one embodiment, the bodily tissue is a biopsy sample.

Examples of bodily fluids include, but are not limited to, blood, plasma, serum, mucus, saliva, urine, sweat, milk, lymph, cerebrospinal fluid, peritoneal fluid, pericardial fluid, pleura fluid, synovial fluid, menstrual fluid, semen and vaginal fluid.

In a preferred embodiment, the bodily tissue or fluid is blood. In a preferred embodiment, the bodily tissue or fluid is brain. In a preferred embodiment, the bodily tissue or fluid is a brain biopsy sample.

In one embodiment, the selected aptamer library comprises from 1 to $10^{11}$ oligonucleotide sequences.

In one embodiment, the selected aptamer library comprises oligonucleotide sequences of 65 to 100 nucleotides in length, preferably of 66 to 99 nucleotides, 67 to 98 nucleotides, 68 to 97 nucleotides, 69 to 96 nucleotides, 70 to 95 nucleotides, 71 to 94 nucleotides, 72 to 93 nucleotides, 73 to 92 nucleotides, 74 to 91 nucleotides, 75 to 90 nucleotides, 76 to 89 nucleotides, 77 to 88 nucleotides, 78 to 87 nucleotides, 79 to 86 nucleotides in length.

In one embodiment, the selected aptamer library comprises oligonucleotide sequences comprising, from 5' to 3':

- a 5' known primer recognition sequence region,
- a random region, and
- a 3' known primer recognition sequence region.

In one embodiment, the random region comprises a sequence from 20 to 60 nucleotides in length, preferably from 22 to 58, from 24 to 56, from 26 to 54, from 28 to 52, from 30 to 50, from 32 to 48, from 34 to 46, from 36 to 44, from 38 to 42. In one embodiment, the random region comprises a sequence of 40 nucleotides in length.

In one embodiment, the known primer recognition sequence region comprises a sequence from 10 to 35 nucleotides in length, preferably from 12 to 33, from 14 to 31, from 16 to 29, from 18 to 27, from 20 to 25. In one embodiment, the random region comprises a sequence of 23 nucleotides in length.

In one embodiment, the known primer recognition sequence region is selected from sequences with SEQ ID NO: 47 and 48.

SEQ ID NO: 47 AACTACATGGTATGTGGTGAACT
SEQ ID NO: 48 GACGTACAATGTACCCTATAGTG

An example of oligonucleotide sequence is given as SEQ ID NO: 1:
5'AACTACATGGTATGTGGTGAACT($N_{40}$)GACGTACAATGTACCCTATAGTG-3' (SEQ ID NO: 1), where N can be A, T, C, G, U or any modified nucleotide, preferably N can be A, T, C or G.

In one embodiment, the frequency of each oligonucleotide sequence in the selected aptamer library ranges from about 1 to about 1.000.000.

In one embodiment, the bodily tissues or fluids were previously taken from the reference subject, *i.e.,* the uses and methods of the present invention do not comprise a step of recovering said bodily tissues or fluids from the reference subject. Consequently, according to this embodiment, the uses and methods of the present invention are non-invasive uses and methods, *i.e., in vitro* uses and methods.

As used herein, the term "**reference subject**" refers to a subject with known outcome for the medical state, disease or condition under investigation, *i.e.,* a subject who is/was diagnosed with the medical state, disease or condition under investigation by medical diagnosis, including but not limited to, differential diagnosis, pattern recognition, diagnostic criteria, clinical decision support-based system, medical algorithm, diagnostic workup, sensory pill, optical coherence tomography and any use and method of the present invention.

In one embodiment, the reference subject is a mammal, preferably a primate, more preferably a human. In one embodiment, the reference subject is a man. In one embodiment, the reference subject is a woman. In one embodiment, the reference subject is above the age of 20, preferably above the age of 30, 40, 50, 60, 70, 80, 90 years old or more. In one embodiment, the reference subject is from 30 to 90 years old, preferably from 40 to 90 years old, more preferably from 50 to 90 years old, even more preferably from 60 to 90 years old, even more preferably from 70 to 90 years old.

In one embodiment, the bodily tissue or fluid is provided from a substantially healthy reference subject. As used herein, the term "**substantially heathy**" refers to a subject who is/was not diagnosed with the medical state, disease or condition under investigation. In one embodiment, the bodily tissue or fluid is provided from a reference subject who is/was diagnosed with the medical state, disease or condition under investigation.

In one embodiment, the selected aptamer library is prepared by reiterative selection of a random set of oligonucleotide sequences against a pool of bodily tissues or fluids from several reference subjects. In one embodiment, the selected aptamer library is prepared by reiterative counter-selection of a random set of oligonucleotide sequences against a pool of bodily tissues or fluids from several reference subjects. In one embodiment, the selected aptamer library is prepared by alternating reiterative selection and reiterative counter-selection of a random set of oligonucleotide sequences against a pool of bodily tissues or fluids from several reference subjects.

As used herein, the term "**pool of bodily tissues or fluids**" refers to a sample comprising a mixture of bodily tissues or fluids provided from at least 2, preferably at least 3, 4, 5, 6, 7, 8, 9, 10, 12, 14, 16, 18, 20, 25, 50 or more reference subjects. In one embodiment, the pool of bodily tissues or fluids comprises similar or identical tissues or fluids in nature, provided from at least 2, preferably at least 3, 4, 5, 6, 7, 8, 9, 10, 12, 14, 16, 18, 20, 25, 50 or more reference subjects. In one embodiment, the pool of bodily tissues or fluids is provided from substantially healthy reference subjects. In one embodiment, the pool of bodily tissues or fluids is provided from reference subjects who are/were diagnosed with the medical state, disease or condition under investigation.

In a preferred embodiment, the pool of bodily tissues or fluids comprises the same type of biological sample from several individuals, all affected by the same medical state, disease or condition.

In a preferred embodiment, the selected aptamer library is prepared by reiterative selection against a pool of the same type of bodily tissues or fluids from several individuals affected with the medical state, disease or condition under investigation.

In a preferred embodiment, the selected aptamer library is prepared by reiterative selection against a pool of the same type of bodily tissues or fluids from several individuals not affected with the medical state, disease or condition under investigation.

It is well-known in the art that selection represents retention of oligonucleotide sequences that bind to the biological sample (*i.e.,* to the bodily tissues or fluids of pool thereof) and counter-selection represents retention of oligonucleotide sequences that do not bind to the biological sample (*i.e.,* to the bodily tissues or fluids of pool thereof).

In one embodiment, the selected aptamer library is maintained at least 1 month, at least 3 months, at least 6 months, at least a year, at least 5 years, at least 10 years, preferably indefinitely.

In one embodiment, the selected aptamer library is maintained through PCR amplification.

In one embodiment, the selected aptamer library is maintained without diagnostic selection.

In one embodiment, the selected aptamer library is maintained by chemical synthesis using techniques known in the art.

In one embodiment, a subset of aptamers sequences can be identified within the selected aptamer library and synthesized. As used herein, such aptamer sequences are termed "**AptaMarkers**". In one embodiment, AptaMarkers can be combined to form a "**simulated selection aptamer library**", wherein the simulated selection aptamer library comprises known AptaMarkers at known relative frequencies.

In one embodiment, the uses and methods of the present invention comprise a further step of analyzing the selected aptamer library. In one embodiment, analyzing the selected aptamer library comprises determining the nature, amount and/or relative frequency of each aptamer sequence. In one embodiment, analyzing the selected aptamer library comprises subjecting said library to next generation sequencing (NGS), qPCR, antisense sequence hybridization or quantitative ligase chain reaction (qLCR).

In a preferred embodiment, the selected aptamer library is subjected to NGS analysis. The copy number of each sequence captured by NGS analysis from the selected aptamer library is determined.

In one embodiment, the copy number of each aptamer sequence observed in the selected aptamer library is determined. In one embodiment, the relative copy number of each aptamer sequence observed in the selected aptamer library is determined. In one embodiment, the relative copy number of each aptamer sequence observed in the selected aptamer library is determined by dividing the copy number by the total number of aptamer sequences observed in said library.

In one embodiment, the relative frequency of each aptamer sequence observed in the selected aptamer library is determined by dividing the observed number of each aptamer sequence by the total number of sequences observed for that aptamer library.

In one embodiment, the uses and methods of the present invention comprise a further step of contacting the selected aptamer library with a biological sample from at least one subject. In one embodiment, the uses and methods of the present invention comprise a further step of contacting the selected aptamer library with a biological sample from at least two subjects. In one embodiment, the uses and methods of the present invention comprise a further step of contacting the selected aptamer library with a biological sample from at least 3, 4, 5, 6, 7, 8, 9, 10, 11, 12, 13, 14, 15, 16, 17, 18, 19, 20 subjects or more.

In one embodiment, the subject is/was diagnosed with the medical state, disease or condition under investigation. In one embodiment, the subject is at risk of developing the medical state, disease or condition under investigation. In one embodiment, the subject is/was not diagnosed with the medical state, disease or condition under investigation. In one embodiment, the at least two subjects or more are/were diagnosed with the medical state, disease or condition under investigation. In one embodiment, the at least two subjects or more are at risk of developing the medical state, disease or condition under investigation. In one embodiment, the at least two subjects or more are/were not diagnosed with the medical state, disease or condition under investigation. In one embodiment, the at least two subjects or more comprise at least one subject who is/was diagnosed with the medical state, disease or condition under investigation and at least one subject who is/was not diagnosed with the medical state, disease or condition under investigation. In one embodiment, the at least two subjects or more are/were diagnosed with the medical state, disease or condition under investigation and exhibit different stage or phase of the medical state, disease or condition under investigation.

In one embodiment, the uses and methods of the invention comprise a further step of contacting the selected aptamer library with a biological sample from a "diagnostic subject". The term "**diagnostic subject**" as used herein refers to a subject being investigated for a medical state, disease or condition.

In one embodiment, the subject is/was diagnosed with the medical state, disease or condition under investigation. In one embodiment, the subject is at risk of developing the medical state, disease or condition under investigation. In one embodiment, the subject is/was not diagnosed with the medical state, disease or condition under investigation. Preferably as used herein, the "diagnostic subject" is at risk of developing and/or is/was not diagnosed with the medical state, disease or condition under investigation.

In one embodiment, the subject or diagnostic subject is a mammal, preferably a primate, more preferably a human. In one embodiment, the subject or diagnostic subject is a man. In one embodiment, the subject or diagnostic subject is a woman. In one embodiment, the subject or diagnostic subject is above the age of 20, preferably above the age of 30, 40, 50, 60, 70, 80, 90 years old or more. In one embodiment, the subject or diagnostic subject is from 30 to 90 years old, preferably from 40 to 90 years old, more preferably from 50 to 90 years old, even more preferably from 60 to 90 years old, even more preferably from 70 to 90 years old.

In one embodiment, the medical state, disease or condition include, but are not limited to, neurodegenerative diseases, neurological diseases, cancers, autoimmune diseases, cardiovascular diseases, infections, inflammatory diseases and metabolic diseases.

Examples of neurodegenerative diseases include, but are not limited to, Alzheimer's disease, mild cognitive impairment (MCI), impaired memory functions, Parkinson's disease, Huntington's disease, multiple sclerosis, amyotrophic lateral sclerosis (ALS) (including familial ALS and sporadic ALS), Pick's disease, dementia, depression, sleep disorders, psychoses, epilepsy, schizophrenia, paranoia, attention deficit hyperactivity disorder (ADHD), amnesiac syndromes, progressive supranuclear palsy, brain tumor, head trauma and Lyme disease.

In a preferred embodiment, the medical state, disease or condition is Alzheimer's disease.

In one embodiment, the subject or diagnostic subject is not receiving medication for the medical state, disease or condition. In one embodiment, the subject or diagnostic subject is not receiving medication for Alzheimer's disease.

In one embodiment, the subject or diagnostic subject is receiving medication for the medical state, disease or condition. In one embodiment, the subject or diagnostic subject is receiving medication for Alzheimer's disease.

Examples of Alzheimer's disease medications include, but are not limited to, acetylcholinesterase inhibitors, NMDA receptor antagonists and antibodies.

Examples of acetylcholinesterase inhibitors include, but are not limited to, donepezil (Aricept®, Namzaric®), rivastigmine (Exelon®), galantamine (Razadyne®), huperzine A and tacrine (Cognex®).

Examples of NMDA receptor antagonists include, but are not limited to, memantine (Axura®, Ebixa®, Namenda®, Namzaric®).

Examples of antibodies include, but are not limited to, monoclonal antibodies directed against A$\beta$ (such as,

e.g., aducanumab, bapineuzumab, crenezumab, gantenerumab, ponezumab, solanezumab) and immunoglobulin therapies (such as, *e.g.*, Gammagard®, Flebogamma®).

In one embodiment, the Alzheimer's disease medication is an agency-approved medication, *i.e.,* a medication which has been approved by a national or regional drug agency selected from the group consisting of Food and Drug Administration (FDA - United States), European Medicines Agency (EMA - European Union), Pharmaceuticals and Medical Devices Agency (PMDA - Japan), China Food and Drug Administration (CFDA- China) and Ministry of Food and Drug Safety (MFDS - South Korea).

In one embodiment, the selected aptamer library is aliquoted into subsamples. In one embodiment, each selected aptamer library subsample is contacted with the same type of biological sample (*i.e.,* of bodily tissue or fluid from the diagnostic subject) than that from the reference subjects used for the initial selection when preparing the selected aptamer library. In other words, each selected aptamer library subsample is contacted with a biological sample from an individual subject. That is, the selected aptamer library is applied in a selection process to several different individual biological samples (*i.e.,* bodily tissue or fluid). The number of individuals sampled in this manner is not limited.

In one embodiment, the biological samples (*i.e.*, bodily tissue or fluid from the diagnostic subject) are processed on an ongoing basis over time as patient samples are received.

In one embodiment, the uses and methods of the present invention further comprise a step of obtaining at least one subject-specific aptamer library. In one embodiment, the uses and methods of the present invention further comprise a step of obtaining at least two subject-specific aptamer libraries. In one embodiment, the uses and methods of the present invention further comprise a step of obtaining at least 3, 4, 5, 6, 7, 8, 9, 10, 11, 12, 13, 14, 15, 16, 17, 18, 19, 20 or more subject-specific aptamer libraries.

In one embodiment, obtaining a subject-specific aptamer library comprises selecting aptamers from the selected aptamer library that bind to the subject's or diagnostic subject's biological sample.

As used herein, the aptamer library derived from such a selection is referred to as a "**subject-specific aptamer library**" or "**patient-specific aptamer library**", and the selection is referred to as a "**diagnostic selection**".

In one embodiment, the subject-specific aptamer library comprises aptamer sequences specific for at least one epitope, at least 2, at least 3, at least 4, at least 5, at least 10, at least 20, at least 30 or more epitopes, preferably pathological epitopes.

In one embodiment, the subject-specific aptamer library is obtained by selection of oligonucleotide sequences of a selected aptamer library as described hereinabove, against a bodily tissue or fluid from a subject or diagnostic subject. In one embodiment, the diagnostic selection comprises a single round of selection against a bodily tissue or fluid from a subject or diagnostic subject.

In one embodiment, the subject-specific aptamer library is obtained by reiterative selection of oligonucleotide sequences of a selected aptamer library as described hereinabove, against a bodily tissue or fluid from a subject or diagnostic subject. In one embodiment, the reiterative diagnostic selection comprises at least one round, at least 2, 3, 4, 5, 6, 7, 8, 9, 10, 11, 12, 13, 14, 15, 16, 17, 18, 19, 20, 25, 30, 35, 40, 50 rounds or more of selection against a bodily tissue or fluid from a subject or diagnostic subject.

In one embodiment, the subject-specific aptamer library comprises from 1 to $10^{11}$ oligonucleotide sequences.

In one embodiment, the subject-specific aptamer library comprises oligonucleotide sequences of 65 to 100 nucleotides in length, preferably of 66 to 99 nucleotides, 67 to 98 nucleotides, 68 to 97 nucleotides, 69 to 96 nucleotides, 70 to 95 nucleotides, 71 to 94 nucleotides, 72 to 93 nucleotides, 73 to 92 nucleotides, 74 to 91 nucleotides, 75 to 90 nucleotides, 76 to 89 nucleotides, 77 to 88 nucleotides, 78 to 87 nucleotides, 79 to 86 nucleotides in length.

In one embodiment, the subject-specific aptamer library comprises oligonucleotide sequences comprising, from 5' to 3':

- a 5' known primer recognition sequence region,
- a random region, and
- a 3' known primer recognition sequence region.

In one embodiment, the random region comprises a sequence from 20 to 60 nucleotides in length, preferably from 22 to 58, from 24 to 56, from 26 to 54, from 28 to 52, from 30 to 50, from 32 to 48, from 34 to 46, from 36 to 44, from 38 to 42. In one embodiment, the random region comprises a sequence of 40 nucleotides in length.

In one embodiment, the known primer recognition sequence region comprises a sequence from 10 to 35 nucleotides in length, preferably from 12 to 33, from 14 to 31, from 16 to 29, from 18 to 27, from 20 to 25. In one embodiment, the random region comprises a sequence of 23 nucleotides in length.

In one embodiment, the known primer recognition sequence region is selected from sequences with SEQ ID NO: 47 and 48.

SEQ ID NO: 47 AACTACATGGTATGTGGTGAACT

SEQ ID NO: 48 GACGTACAATGTACCCTATAGTG

An example of oligonucleotide sequence is given as SEQ ID NO: 1: 5'AACTACATGGTATGTGGTGAACT($N_{40}$)GACGTACAATGTACCCTATAGTG-3' (SEQ ID NO: 1), where N can be A, T, C, G, U or any modified nucleotide, preferably N can be A, T, C or G.

In one embodiment, the frequency of each oligonucleotide sequence in the subject-specific aptamer library ranges from about 1 to about 1.000.000.

In one embodiment, the uses and methods of the present invention comprise a further step of analyzing the subject-specific aptamer library. In one embodiment, analyzing the subject-specific aptamer library comprises determining the nature, amount and/or relative frequency of each aptamer sequence. In one embodiment, analyzing the subject-specific aptamer library comprises subjecting said library to next generation sequencing (NGS), qPCR, antisense sequence hybridization or quantitative ligase chain reaction (qLCR).

In a preferred embodiment, the subject-specific aptamer library is subjected to NGS analysis. The copy number of each sequence captured by NGS analysis from the subject-specific aptamer library is determined.

In practice, a selected aptamer library would be applied in diagnostic selection on individual biological samples (*i.e.*, bodily tissue or fluid) from a subject or diagnosis subject. The subject-specific aptamer library would then be subjected to NGS analysis and the relative frequency of the aptamer sequences within the library would be determined. In one embodiment, the relative frequency of diagnostic aptamers would be determined through a method other than NGS analysis, such as quantitative PCR analysis, hybridization to antisense sequences, or quantitative LCR analysis.

In one embodiment, the copy number of each aptamer sequence observed in the subject-specific aptamer library is determined. In one embodiment, the relative copy number of each aptamer sequence observed in the subject-specific aptamer library is determined. In one embodiment, the relative copy number of each aptamer sequence observed in the subject-specific aptamer library is determined by dividing the copy number by the total number of aptamer sequences observed in said library. In one embodiment, the relative frequency of each aptamer sequence observed in the subject-specific aptamer library is determined by dividing the observed copy number of a given aptamer by the total number of aptamers observed in that library.

In one embodiment, the uses and methods of the present invention further comprise a step of determining the change in relative frequency between aptamers from the selected aptamer library and the subject-specific aptamer library.

In one embodiment, the uses and methods of the present invention further comprise a step of determining the change in relative frequency between aptamers from at least two subject-specific aptamer libraries.

The relative frequency of an aptamer in a library is a function of the selection process on the entire system of aptamers. Thus, the change in relative frequency of an aptamer is a function of the selection on the entire library rather than on the aptamer by itself.

It is understood that a selected aptamer library may contain sequences that were not observed in a sequencing analysis, and that the presence of these sequences in the library is relevant to the change in frequency of any aptamer in any given subject-specific aptamer library. It is contemplated that this invention could also be enabled by a subset of the selected aptamer library wherein only known and characterized aptamer sequences were combined at known ratios.

In one embodiment, the relative frequencies of the aptamer sequences within the subject-specific aptamer library would be compared to the relative frequencies of the aptamer sequences in the selected aptamer library, thereby determining the change in aptamer relative frequency. In one embodiment, the relative frequencies of the aptamer sequences within the subject-specific aptamer library would be compared to the relative frequencies of the aptamer sequences in at least one other, at least 2 other, 3, 4, 5, 6, 7, 8, 9 or 10 or more other subject-specific aptamer libraries, thereby determining the change in aptamer relative frequency. In one embodiment, the change in aptamer frequency is a biomarker measurement.

In one embodiment, the change in aptamer relative frequency for each aptamer sequence observed in both the selected aptamer library and the subject-specific aptamer library is determined by dividing the relative frequency of each subject-specific aptamer library's aptamer by the relative frequency of the same aptamer in the selected aptamer library. In one embodiment, the change in aptamer relative frequency for each aptamer sequence observed in at least two subject-specific aptamer libraries is determined by dividing the relative frequency of a subject-specific aptamer library's aptamer by the relative frequency of the same aptamer in another subject-specific aptamer library.

In one embodiment, the comparison of the relative frequencies comprises co-variance characterization.

Many methods are known in the art for characterizing co-variance. Examples of such methods, such as correlation analysis, are provided in the Examples of the present application.

Thus, in one embodiment, the uses and methods of the present invention comprise a step of correlating changes in relative frequency between aptamers from the selected aptamer library and the subject-specific aptamer library to the medical state, disease or condition of the at least one subject. In one embodiment, the uses and methods of the present invention comprise a step of correlating changes in relative frequency between aptamers within subject-specific aptamer libraries to the medical state disease or condition of the at least two subjects.

In one embodiment, analysis of covariance of aptamer relative frequencies is used to determine which aptamers co-vary across reference and diagnostic subjects, and which aptamers do not co-vary across reference and diagnostic subjects. In another embodiment, analysis of covariance of aptamer relative frequencies is used to determine which aptamers co-vary across subjects of known outcome and diagnostic subjects, and which aptamers do not co-vary across subjects of known outcome and diagnostic subjects. It is a reasonable assumption that those aptamers that co-vary are binding to the same pathological entity and those that do not co-vary are not binding to the same pathological entity.

In one embodiment, co-variance of the change in relative frequency among the aptamers is evaluated through a standard co-variance analysis process across multiple subject-specific aptamer libraries.

In one embodiment, aptamers are clustered relative to each based on the co-variance analysis.

In one embodiment, representative aptamers from each aptamer cluster are used to characterize differences among subjects of known outcome.

In one embodiment, a comparison of the relative frequencies is made between samples that are from patients known to be suffering from the disease, preferably from an advanced stage of the disease, and samples that are from individuals that are not affected by the pathology. This information is used to define which aptamers have diagnostic relevance. These aptamers are named "**AptaMarkers**".

In one embodiment, a distance matrix is derived from the correlation values to enable the visualization of the clusters of co-varying aptamers.

With the inclusion of biological samples from individuals not affected by the pathology (*i.e.,* of bodily tissue or fluid from substantially healthy reference subjects), it is possible to identify those clusters of pathological aptamers that provide a consistent division between individuals affected by a pathology and those unaffected by the pathology. The analysis of the change in aptamer frequency provides the basis for a diagnosis of the pathology through identical analysis of a biological sample from a diagnostic subject whose medical state is unknown.

Moreover, it is demonstrated that certain pathological entities may not be shared by all individuals affected by a pathology. The relative abundance of such pathological entities can be characterized for individuals by analyzing the co-variance of a cluster of aptamers that define a pathological entity across individuals.

The characterization of variation in the relative abundance of pathological entities may be of value in determining the identity of such entities.

In one embodiment, the uses and methods of the present invention further comprise a step of applying the selected aptamer library to subjects for which the medical state, disease or condition is unknown (herein termed as "**diagnostic subjects**"), thereby providing a diagnosis based on the correlations determined between the relative change of aptamers within the library and said medical state, disease or condition.

Future use of these biomarkers would involve subsequent application of the selected aptamer library to biological samples from other subjects for diagnosis. One embodiment of this invention is to synthesize the most frequent sequences in the selected aptamer library and to combine them at molar ratios equal to what is observed in said selected aptamer library. This would serve as a means of simplifying the selected library and maintaining it indefinitely.

Another embodiment of this invention would involve subsequent application of the selected aptamer library to biological samples from other subjects for diagnosis. One embodiment of this invention is to synthesize those aptamers where variation in their relative frequency has been shown to be correlated or diagnostic of variation in a medical state amount subjects where the medical state has been characterized. Such a subset of aptamers could be combined at molar ratios equal to what was observed in said selected aptamer library. This would serve as a means of simplifying the selected library and maintaining it indefinitely, and as a means of simplifying analysis.

Embodiments of this invention that involve the use of a subset of the aptamers from the selected library are not limited to equal molar mixtures of the aptamers within such subsets, other means of preparing the subset are feasible, but any means of preparation would require validation prior to application as a diagnostic tool.

Another embodiment of this invention would involve normalization of the relative frequency of the sequences in the library in relation to the total number of sequences captured, or the number of PCR reactions required to amplify the library for analysis, or the relative frequency of a subset of the aptamers, or some combination of these normalization methods. The intent of this embodiment is to reduce error in the analysis by correcting for NGS analysis effect on relative frequency estimates of aptamers.

Many types of statistical analyses could be used to evaluate the degree of clustering or the level of relatedness of the aptamers, including but not limited to principal component analysis, k-clustering, Pearson correlation, or Ward.D2 clustering.

**BRIEF DESCRIPTION OF THE DRAWINGS**

Figure 1 is a graph showing the distribution of copy number of aptamers in selection round #16 ("selected library") against brain tissue of patients affected with Alzheimer's disease with counter selection against healthy brain tissue. The y axis describes the observed copy number of each sequence within selection round #16. The x axis describes the frequency with which each observed copy number was observed.

**Figure 2** is a radial neighbor-joining tree diagram showing the covariance analysis of 408 aptamers against seven brain tissue samples from five individuals with Alzheimer's disease, and two healthy brain tissue samples. Although it is difficult in this rendition of the image to discern the individual labels, this is not as important as the general nature of the clusters of sequences.

**Figure 3** is a histogram showing the normalized change in aptamer frequency across brain tissue samples. The aptamers chosen for this analysis are listed in the legend. "H avg" is the average of the healthy samples.

**Figure 4** is a dendrogram illustrating global relationship among patients affected by Alzheimer's disease.

**Figure 5** is a histogram showing the normalized change in frequency between selection round and "patient specific libraries" for set of aptamers designated as Cluster A for Alzheimer's brain tissue analysis. The aptamers used for this analysis are listed in the legend. "H avg" represents the average values for the healthy sample.

**Figure 6** is a dendrogram representing distance between brain tissue samples from individuals affected by Alzheimer's disease for relative abundance of the pathogenic epitope described by Cluster A.

**Figure 7** is a histogram showing the normalized change in frequency between selection round and "patient specific libraries" for set of aptamers designated as Cluster B. The aptamers used for this analysis are listed in the legend. "H avg" represents the average values for the healthy sample.

**Figure 8** is a dendrogram representing distance between brain tissue samples from individuals affected by Alzheimer's disease for relative abundance of the pathogenic epitope described by Cluster B.

**Figure 9** is a dendrogram illustrating clustering of aptamers from blood serum analysis across patient samples affected by Alzheimer's disease.

**Figure 10** is a dendrogram illustrating relationship among 16 core AptaMarkers chosen from blood serum "patient specific libraries" for patients affected by Alzheimer's disease.

**Figure 11** is a histogram showing the comparison of changes in relative frequency between "patient-specific aptamer libraries" and a healthy sample for 16 blood serum AptaMarkers for Alzheimer's disease.

**Figure 12** is a histogram showing the comparison between an Alzheimer's disease score developed based on the differences in change in relative frequency between each patient and a healthy sample for five blood serum AptaMarkers for Alzheimer's disease and the clinical evaluation of each patient.

**Figure 13** is a scatter plot showing the relative frequencies of 10,000 aptamers between two replicated analysis for the same subject (Ach 1). x-axis represents the relative frequencies of the 10,000 aptamers in the first replicate analysis and y-axis represents the relative frequencies of the 10,000 aptamers in the second replicate analysis.

**Figure 14** is a scatter plot showing the reproducibility of the relative frequencies of the top 100 aptamers. This figure provides the comparison of the lowest number of sequences captured for each sample (y-axis) *versus* the regression coefficient obtained for the samples across replicates in terms of relative frequency of the 100 most highly enriched aptamers (x-axis).

**Figure 15** is a histogram showing the relative change in aptamer frequency versus the change in epitope concentration.

**Figure 16** is a histogram showing a replicated analysis (Rep 1 and Rep2) of Aptamarker-based diagnosis of late Alzheimer's disease (AD) in blood serum.

**Figure 17** is a scatter plot showing the relevance of the AptaMarker score as compared to the commonly used cognitive test MMSE (Mini-Mental State Examination) to diagnose Alzheimer's disease.

**Figure 18** is a box plot showing the overall difference between substantially healthy subjects and Alzheimer's disease patients, based on the AptaMarker score.

**Figure 19** is a histogram showing a replicated analysis (Rep 1 and Rep2) of Aptamarker-based diagnosis of late Alzheimer's disease (AD) in blood serum, on randomized AIBL samples.

**Figure 20** is two histograms showing the means values of the two replicates of: (**A**) Figure 16; and (**B**) Figure 19.

## EXAMPLES

The present invention is further illustrated by the following examples.

### Example 1: Brain tissue analysis for Alzheimer's disease

Brain tissue samples were kindly prepared and provided from the Alzheimer's disease brain bank at the Hôpital Pitié Salpêtrière in Paris under the direction of Professor Charles Duyckaerts.
It is not known how many sequences the selection library contains after any given selection round. We were able to observe a total of 2,337,575 random aptamer sequences with NGS analysis of an aliquot of the library following selection round #16.

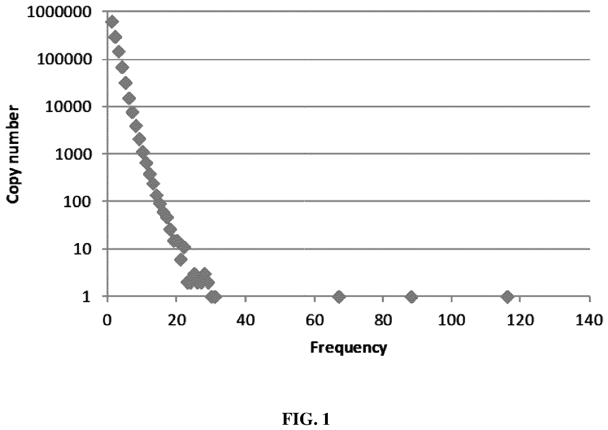

**Figure 1** provides the distribution of copy number of the 2,337,575 sequences observed after selection round #16. We chose a frequency of $5\times10^{-6}$ as the minimum cut-off for inclusion of aptamers after this last selection round in the "selected library" for this example. The distribution of the copy number provides us with a measure of the complexity of the library.
The "selected library" was then used for analysis of individual samples during diagnostic selections. In this example, a first selection round of the selected library of aptamers towards individual brain tissue samples was considered as a "diagnostic selection", and provides a "diagnostic library".
Brain tissue samples used for analysis consisted of PMDc (premotor dorsal, caudal) cerebral cortex brain tissue from five male patients whom had died of Alzheimer's disease (stage VI according to Braak staging; Thai amyloid phase 5; average age of death: 77,2 [from 69 to 87]). The presence of Aβ plaques and tau tangles was confirmed in each sample. One sample was replicated. We also included two separate individual healthy brain tissue samples. These samples do not represent a replication in the same manner as the replication of the pathological sample does, as the two healthy samples were from two different individuals. For analysis, the average of these two healthy samples was used. We chose a frequency of $5\times10^{-6}$ as the minimum cut-off for inclusion of aptamers from the selected library for analysis within the diagnostic selections. This represented 1050 sequences.
Of these 1050 sequences, 408 were observed in at least six of the diagnostic libraries. These 408 sequences were then chosen for further analysis.
We normalized the relative frequency of these 408 aptamers in each library by dividing the individual frequencies by the average frequency for the entire set. We then divided the normalized frequency in each "subject-specific aptamer library" by the normalized frequency in the "selected aptamer library". This provides us with a dataset of normalized change values for each of the 408 aptamers against each of the seven brain tissue samples.
We perform covariance analysis for the aptamers according to the following formula:

$$r = rxy = \frac{1}{n-1}\sum_{i=1}^{n}\left(\frac{xi-\bar{x}}{sx}\right)\left(\frac{yi-\bar{y}}{sy}\right)$$

where:

-   $\bar{x}$ and $\bar{y}$ are the average values for all 408 aptamers within each brain tissue sample,
-   sx and sy are the standard deviations of $\bar{x}$ and $\bar{y}$, and
-   xi and yi are the individual aptamer values.

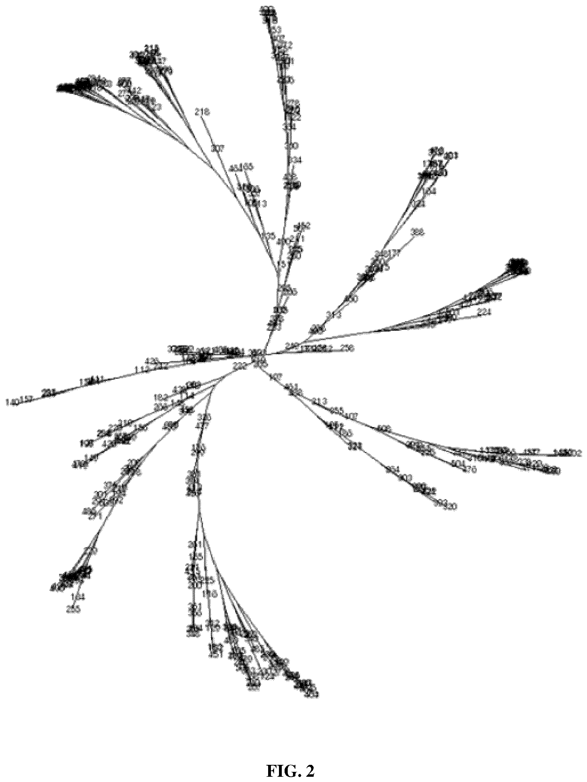

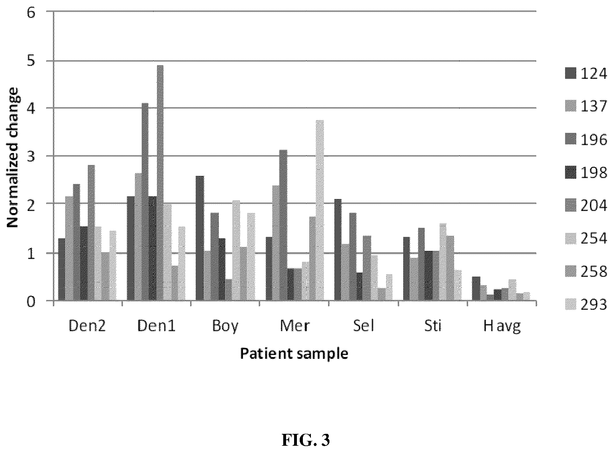

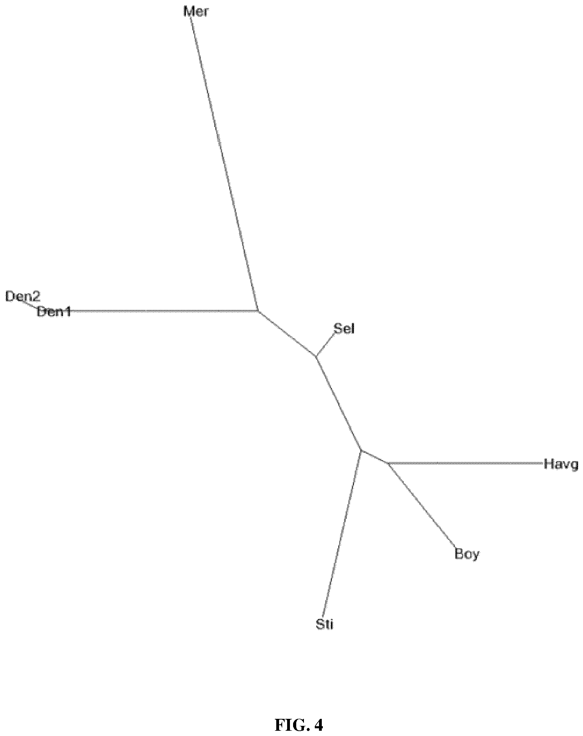

This results in a correlation matrix of 408 by 408 values. We convert this to a distance matrix by subtracting each value from unity. The distance matrix is converted to a Newick formula, which is used to visualize the relationship with the Trex online program (http://www.trex.uqam.ca/index.php?action=matrixToNewick&project=trex).
The overall relationships among all 408 aptamers is provided in **Figure 2**. Clear clustering of aptamers was observed.
Certain of these aptamers were chosen for a demonstration of the diagnostic capacity of this approach across patients. The rate of change data observed for each of these aptamers is provided in **Figure 3** and the spatial relationships following covariance analysis of the patients is provided in **Figure 4.** Covariance analysis of the patients was performed in the same way as covariance analysis of the aptamers, except that the mean and standard deviation values were determined for each biological sample.

It is clear from **Figure 4** that the two replicates of the "Den" sample exhibited the same normalized change from the selected library. It is also clear that the distance of the samples from the healthy sample differed. This should not be taken necessarily as a difference in the severity of the disease. A more reasonable interpretation is that these patients simply exhibit different pathological epitopes for the disease and that certain of them exhibit more such epitopes than others. Indeed, all of the patients suffered from late stage Alzheimer's disease.

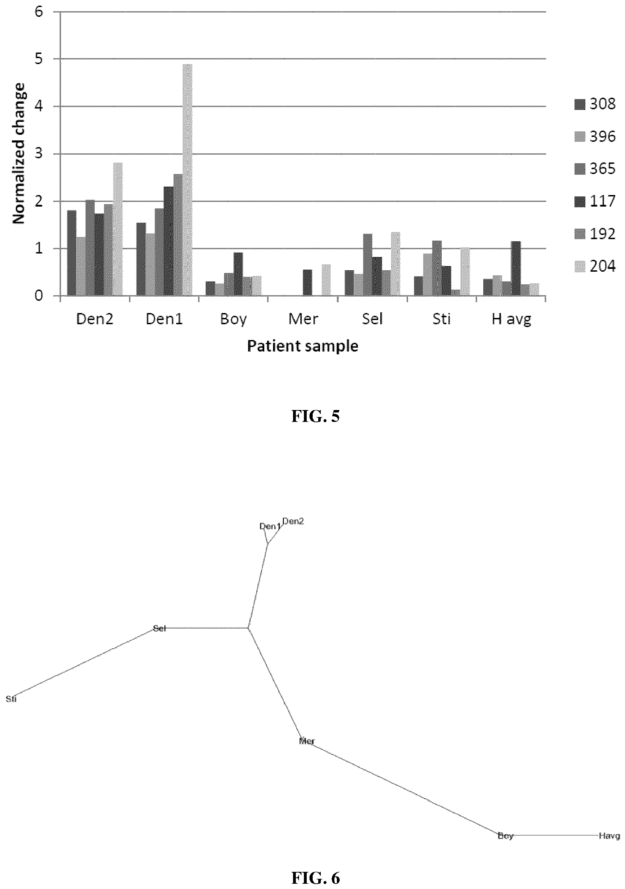

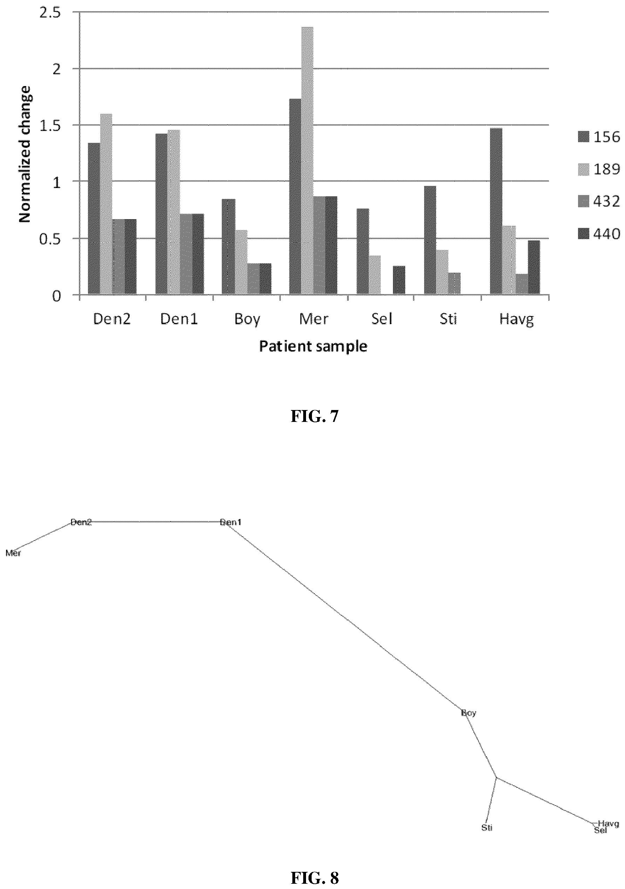

Each cluster is based on different pathological epitopes. This is demonstrated by analyses we performed on different clusters of aptamers. The rates of change for the aptamer cluster A is presented in **Figure 5** and the dendrogram illustrating the relationship among the patients for this pathogenic epitope is presented in **Figure 6.** The same information for the aptamer cluster B is presented in **Figures 7** and **Figure 8** respectively.

For cluster A, patient "Boy" did not exhibit significantly more of this pathogenic epitope than the healthy samples. For cluster B, patient "Sti" did not exhibit significantly more of the pathogenic epitope than the healthy samples. This clearly illustrates that these clusters of aptamers bind to different target entities. This also clearly demonstrates that the patients differ in their relative abundance of pathogenic epitopes. In certain cases, this variation represents a lack of difference between the affected patient and healthy individuals for a specific pathogenic epitope.

**Example 2: Blood serum analysis for Alzheimer's disease**

Patient samples were kindly provided from the IFRAD cohort at the Hôpital Pitié Salpêtrière in Paris. These were derived from patients whom had been evaluated for Alzheimer's disease by a team of clinicians at the hospital led by Professor Bruno Dubois. Patients labeled P1 to P3 and P9 exhibited late stage Alzheimer's disease. Patients labeled P4 to P8 exhibited symptoms of mid to early Alzheimer's disease. Healthy blood serum samples were purchased from Sigma Aldrich Chemicals.

Reiterative selection of a DNA aptamer library identical in description to the library used in Example 1 was used for this example. The proprietary aptamer selection process called FRELEX was used throughout this analysis (International patent application WO2017035666) under license from NeoVentures Biotechnology in Canada. This process circumvents any need for immobilization of target molecules. The library was subjected to 23 rounds of reiterative selection against a pool of blood serum from all four late AD patients. Counter selection was performed against a healthy blood serum sample purchased from Sigma Aldrich.

Selection rounds #16 to #23 were analyzed by next generation sequence (NGS) analysis. A composite set of 400 aptamer sequences were chosen on the basis of high copy number and conservation from one selection to the next.

The "selected library" from selection round #23 was aliquoted and applied to selection against blood serum from each of the nine patients individually, as well as two replicates of the healthy blood serum. Each selection was repeated but for patient samples 6 and 7, for which an insufficient amount of library was recovered after the first selection round to enable a second selection. These selected libraries were designated as "patient specific libraries" and all were subjected to NGS analysis.

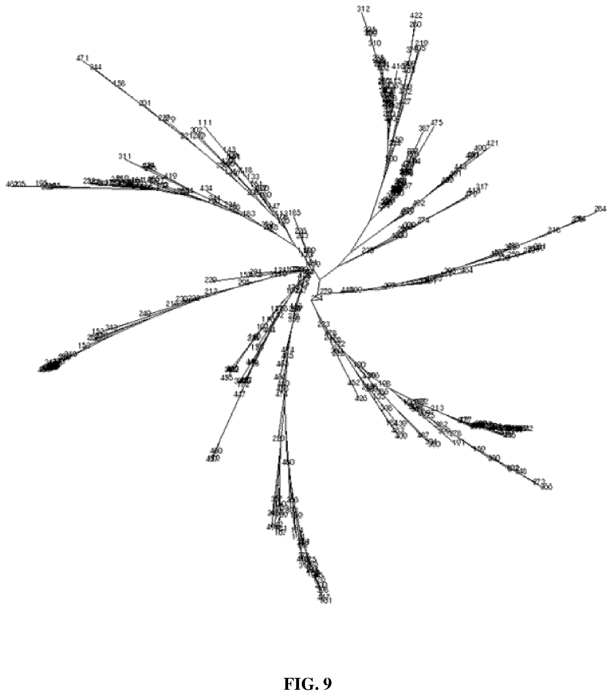

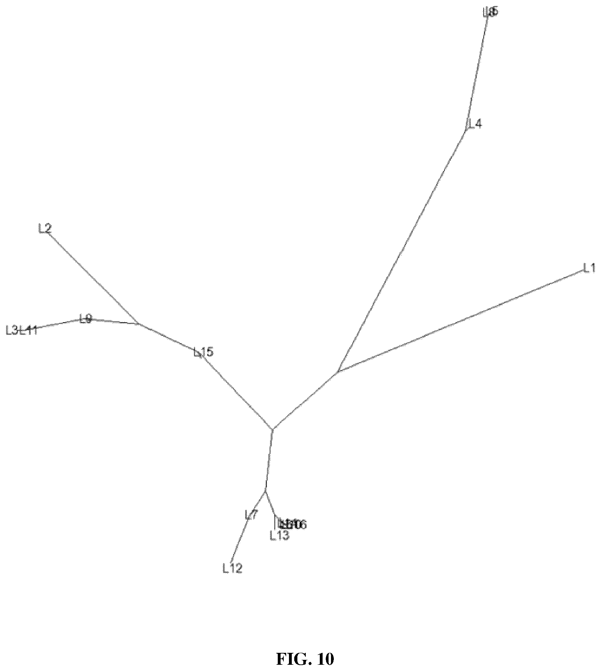

A covariance analysis of the top 400 sequences across the four late AD patients and the two healthy patients is presented in **Figure 9.** The correlation analysis was performed on the change in relative frequency between each of these "patient specific libraries" and the "selected library" (*i.e.*, the library from selection round #23). It is clear that a large number of clusters of sequences was observed, indicating that our aptamer libraries bound to several different pathological epitopes in the blood serum. A subset of sixteen of these sequences was chosen on the basis of representing the range of epitopes characterized by the set of 400 aptamers. A covariance analysis of this subset of 16 aptamers against the four late AD patients and the two healthy library samples is provided in **Figure 10.** We call these 16 aptamers, "AptaMarkers".

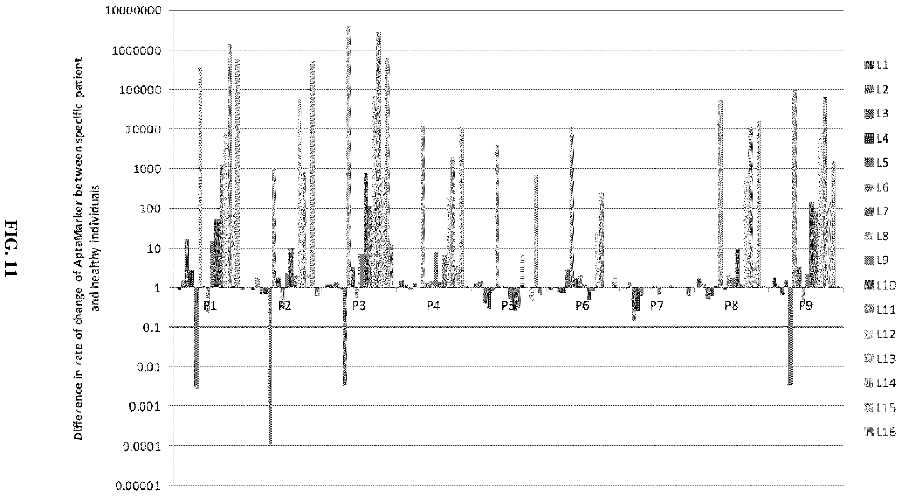

The key for diagnosis with blood serum is the comparison between changes in relative frequency between patients with Alzheimer's disease and healthy individuals. In **Figure 11,** we provide a graphic fingerprint of the relative frequency of each of the sixteen AptaMarkers in each of nine Alzheimer's disease patients divided by the relative frequency in the healthy sample. The clinical score attributed to each of these patients is provided in **Table 1.** The lower the clinical score value, the greater the severity of the disease, a maximum score of 30 indicating an individual not affected by the disease.

**Table 1:** Clinical scores for nine Alzheimer's disease patients used in this example.

| Patient | Clinical score (MMS) |
|---------|---------------------|
| P1 | 0 |

(continued)

| Patient | Clinical score (MMS) |
|---|---|
| P2 | 1 |
| P3 | 2 |
| P4 | 19 |
| P5 | 18 |
| P6 | 19 |
| P7 | 25 |
| P8 | 19 |
| P9 | 3 |

This is a useful means of portraying the differences, as it removes the scale of the change in relative frequency and replaces it with the scale of the difference between the late Alzheimer's disease patients and the healthy sample.

Those AptaMarkers that exhibit a large increase in the late Alzheimer's disease patients presumably represent epitopes that are enriched in the late Alzheimer's disease patients relative to the healthy patients.

It is interesting to note that these differences are not entirely consistent across the patients. That is, certain AptaMarkers show striking differences between certain patients and the healthy sample, while the same difference is not evident for the same AptaMarker with other patients. For instance, the AptaMarker L3 is not significantly different from the healthy sample for patient 1, while it is for patient 2 and 3. The AptaMarker L14 does not exhibit a significant difference from the healthy sample for patient 2, while it does for patients 1 and 3. The AptaMarker L16 only exhibits a significant difference between patient 3 and the healthy sample, and not for the other two patients. This is presumably due to differences among the patients for the epitopes that they exhibit as a result of their pathology. This observation leads us to conclude that to enable adequate diagnosis of Alzheimer's disease, it is necessary to employ several AptaMarkers.

A key application of the present invention is the use of this method to diagnose Alzheimer's disease in a patient. This would be accomplished by demonstrating that the enrichment rate between a "diagnostic library" and the "selected library" for at least two AptaMarkers is significantly greater than the enrichment rate for these same two AptaMarkers on healthy samples.

It is also clear in **Figure 11** that the patients exhibiting less progression of the pathology based on clinical scores (MMI) also exhibit less difference in change in relative frequency of AptaMarkers as compared to a healthy sample.

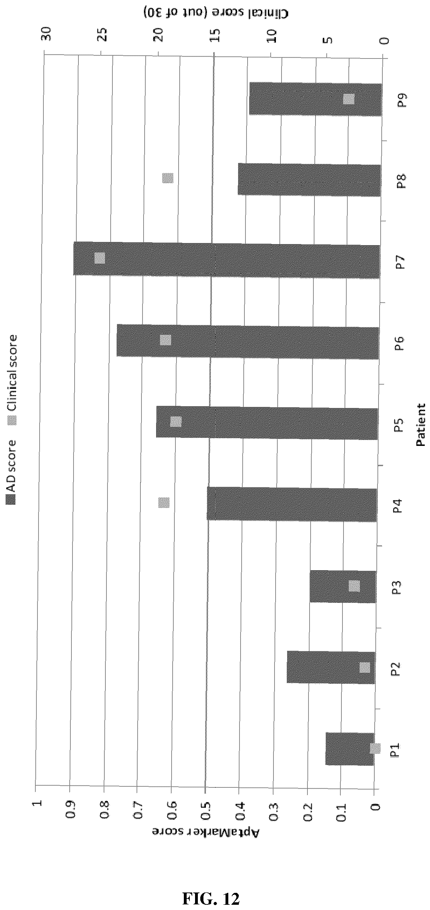

We developed an algorithm based on the relative proportion of five of the AptaMarkers portrayed in **Figure 11** (L2, L6, L12, L13 and L15) of the maximum value observed across the nine patients for each AptaMarker as a log(10) of the values displayed. The average of these relative proportions was determined for each patient as an AD score. The clinical assessment value for each patient is provided with the AD score in **Figure 12.** Over all the patients, we observed a correlation of 0.78 between the AD score and the clinical score. The statistical significance of this correlation is $P > 0.98$, given 8 degrees of freedom.

**Example 3: Blood serum analysis from an extended set of human subjects**

*Materials and Method*

The ssDNA library for selection was composed of a 40-mer random region flanked by two constant regions for primer hybridization (Trilink Biotechnologies, San Diego, CA, USA) as set forth in SEQ ID NO: 1: 5'AACTACATGGTATGTGGTGAACT$(N_{40})$GACGTACAATGTACCCTATAGTG-3' (SEQ ID NO: 1), where N can be A, T, C or G.

Primers used for amplification were purchased from IDT DNA technologies.

The cohort recruitment process including the neuropsychological, lifestyle, and mood assessments have been previously described in detail (Ellis et al., Int Psychogeriatr. 2009 Aug;21(4):672-87). In brief, the AIBL study recruited a total of 1166 participants over the age of 60 years at baseline, of whom 54 were excluded because of comorbid disorders or consent withdrawal. Using the NINCDS-ARDA international criteria for AD diagnosis (Tierney et al., Neurology. 1988 Mar;38(3):359-64) and symptomatic predementia phase criteria for MCI diagnosis (Albert et al., Alzheimers Dement. 2011 May;7(3):270-9), a clinical review panel determined disease classifications at each assessment time point to ensure accurate and consistent diagnoses among the participants. According to these diagnostic criteria, participants were classified into one of three groups; AD (Alzheimer's disease), MCI (mild cognitive impairment), or HCs (healthy controls). At baseline, there were a total of 768 HCs, 133 MCI, and 211 AD subjects.

The AIBL study is a prospective, longitudinal study, following participants at 18-month intervals. This particular study reports on 711 individuals who completed the full study assessment and corresponding blood sample collection at baseline, 18 months and 36 months follow-up time points.

The institutional ethics committees of Austin Health, St. Vincent's Health, Hollywood Private Hospital, and Edith Cowan University granted ethics approval for the AIBL study. All volunteers gave written informed consent before participating in the study.

The samples analyzed in this study are described in the **Table 2.**

| Neo ID | Age | MMSE | | Sample date (in months) | | PET Scan / Amyoloid | Library training | Individual analysis |
|---|---|---|---|---|---|---|---|---|
| | | Sample 1 | Sample 2 | Sample 1 | Sample 2 | | | |
| Ach-1 | 83 | 24 | 0 | 0 | 18 | Positive | | S2 |
| Ach-2 | 83 | 25 | 15 | 0 | 18 | Positive | | S2 |
| Ach-3 | 78 | 14 | 0 | 18 | 36 | Positive | S1 | S2 |
| Ach-4 | 62 | 14 | 5 | 0 | 18 | Positive | S2 | S2 |
| Ach-5 | 84 | 15 | 2 | 36 | 54 | Positive | | S2 |
| Ach-6 | | 22 | 9 | 18 | 36 | Positive | S2 | S2 |
| Ach-9 | 74 | 22 | 13 | 18 | 36 | Positive | | S2 |
| Ach-10 | 62 | 16 | 3 | 18 | 36 | Positive | | S2 |
| Ach-11 | 64 | 24 | 12 | 18 | 36 | Positive | | S2 |
| Ach-12 | 62 | 16 | 4 | 18 | 36 | Positive | | S2 |

| | | | | | | | | |
|---|---|---|---|---|---|---|---|---|
| **Ach-13** | 71 | 22 | 11 | 0 | 18 | Positive | | S2 |
| **Ach-15** | 71 | 13 | 4 | 18 | 36 | Positive | S2 | S2 |
| **Ach-16** | 71 | 18 | 9 | 18 | 36 | Positive | S2 | S2 |
| **Ach-17** | 71 | 21 | 11 | 18 | 36 | Positive | S2 | S2 |
| **Ach-18** | 65 | 12 | 0 | 18 | 36 | Positive | S2 | S2 |
| **Ach-44** | 80 | 17 | 16 | 18 | 36 | Positive | | S2 |
| **Ach-19** | 71 | 30 | 30 | 90 | 108 | Negative | S1 | S2 |
| **Ach-20** | 67 | 29 | 29 | 90 | 108 | Negative | | S2 |
| **Ach-21** | 68 | 29 | 30 | 90 | 108 | Negative | | S2 |
| **Ach-22** | 64 | 29 | 30 | 90 | 108 | Negative | S1 | S2 |
| **Ach-23** | 75 | 29 | 30 | 72 | 90 | Negative | | S2 |
| **Ach-24** | 69 | 30 | 30 | 54 | 72 | Negative | | S2 |
| **Ach-25** | 66 | 30 | 29 | 90 | 108 | Negative | | S2 |
| **Ach-26** | 65 | 28 | 30 | 90 | 108 | Negative | S1 | |
| **Ach-27** | 62 | 29 | 30 | 90 | 108 | Negative | S1 | |
| **Ach-28** | 62 | 29 | 30 | 90 | 108 | Negative | S1 | |
| **Ach-29** | 62 | 30 | 30 | 72 | 90 | Negative | | S2 |

**Table 2**

The Mini-Mental State Examination (MMSE) is a cognitive test developed to diagnose dementia (Folstein et al., J Psychiatr Res. 1975 Nov;12(3):189-98). A score of 30 indicates full cognitive capacity, a decrease in the score from this value is correlated with a decrease in cognitive capacity and a later stage of Alzheimer's disease.

Samples were obtained from the subjects at 18-month intervals, MMSE tests were performed at each sampling date for each subject. In **Table 2,** "sample 1" and "sample 2" describe two subsequent sampling dates 18 months apart. The number of months from the subjects' enrollment in the trial is listed for each sampling date under the heading "sampling date". The column headed "age" provides the age of the subjects at the start of the cohort project. The subjects age at the time of sampling is thus provided by adding the months in the sampling date column to the age.

We used a pool of blood serum from six subjects outlined in the column "Library training" for the positive selection (late stage AD), and five subjects for the negative selection (healthy). Samples for the individual analysis are described in the last column. S1 and S2 refer to "sample 1" and "sample 2", *i.e.,* the sampling date of the sample used for training or analysis.

NeoNeuro SAS was kindly permitted to use the FRELEX selection platform (described in International patent application WO2017035666, which is hereby incorporated by reference in its entirety) for this study by NeoVentures Biotechnology Inc. for the selection of aptamer libraries.

FRELEX requires the preparation of an immobilization field comprising a gold chip coated with thiolated random 8 base pair DNA oligonucleotides (thereafter referred to as "8-mers"). The 8-mers were dissolved in 50 $\mu$L of phosphate buffer saline (PBS) (8.0 mM $Na_2PO_4$, 1.4 mM $KH_2PO_4$, 136 mM NaCl, 2.7 mM KCl, pH 7.4) at a concentration of 10 $\mu$M. This solution was incubated at room temperature (RT) for 1 hour on gold surface chip, dimensions $7 \times 10 \times 0.3$ mm (Xantec, Germany). The chip was then air-dried and 50 $\mu$L of a solution comprising thiol-terminated polyethylene glycol (SH-PEG) molecules and incubated for 30 minutes at RT with gentle shaking. This step blocks any remaining gold surface that is not covered with 8-mers. SH-PEG was subsequently added a second time for 16 hours. After that, the SH-PEG solution was removed from the chip and the functionalized gold chip surface was washed with deionized water and air-dried.

In the first phase of FRELEX (hereafter referred to as "Phase I" or "negative selection phase") employed in this study, $10^{16}$ sequences from the random aptamer library described previously (with nucleic acid sequence set forth in SEQ ID NO: 1) were snap-cooled by heating the library to 95°C for 10 minutes followed by immediate immersion in ice bath. These single stranded (ss) DNA sequences were incubated with the functionalized immobilization field (gold chip coated with 8-mers) in 50 $\mu$L of Selection Buffer (10 mM HEPES, 120 mM NaCl, 5 mM $MgCl_2$, 5 mM KCl) for 30 minutes at RT. The remaining solution was removed and discarded. This removes sequences that have too much secondary structure to enable binding to the 8-mers on the surface. The immobilization field was washed twice with 50 $\mu$L of 10X

TE buffer and the remaining bound oligonucleotides were eluted and recovered with two incubations of 15 minutes each in 1 mL of Selection Buffer at 95 °C. These elutates were pooled and purified using the GeneJET PCR Purification Kit (Thermo Fisher Scientific, Germany) as described by the manufacturer and eluted with 25 $\mu$L of deionized water.

In a second phase of FREELEX ("Phase II" or "positive selection phase"), this aptamer library selected for capacity to bind to 8-mers was used for positive selection with a pool of blood serum from late AD in a total volume of 50 $\mu$L 1X Selection Buffer. This solution was incubated for 15 minutes, then incubated with the immobilization field for 15 minutes at RT. The remaining solution was recovered carefully and stored in a fresh tube. The immobilization field was washed twice with 50 $\mu$L of 1X Selection Buffer with each wash being collected and pooled with the solution removed in the first step. This solution comprises sequences that did not bind to the immobilization field, presumably because they are bound to some moieties within the blood serum instead. This pooled solution was purified as described for "Phase I" of FREELEX, eluted in 400 $\mu$L and subjected to PCR amplification for an appropriate number of cycles to create a clear band of approximately 5 ng of amplified DNA.

After the first round of selection (*i.e.*, Phases I and II), PCR was used to amplify the selected ssDNA into double stranded DNA (dsDNA) for an appropriate number of cycles to create a clear band of approximately 5 ng of amplified DNA. All PCR procedures were carried out according to standard molecular biology protocols and under the following conditions:

- 95°C for 5 minutes,
- x cycles at 95°C for 10 seconds,
- 55°C for 15 seconds,
- 72°C for 30 seconds,
- followed by a final extension at 72°C for 5 minutes.

In this process, we used the PCR primers to create a T7 promoter on the 3' end of the amplified product. Then, the dsDNA was used as a template for *in vitro* transcription to obtain antisense RNA copies using T7 RNA polymerase in an overnight reaction at 55°C. The transcribed antisense RNA was treated with DNase I, purified with RNeasy Minelute cleanup (Qiagen) to remove remaining dsDNA template. This antisense RNA library was then used as the template in a reverse transcription reaction to obtain sense strand cDNA oligonucleotides. The cDNA-RNA mixture was treated with RNase H according to manufacturer instructions to remove remaining antisense RNA molecules. The cDNA was purified in a PCR cleanup column and used as template for the next round of selection.

The selection process described herein above was then reiterated for several rounds. Subsequent selection rounds were performed in the same manner with the exception that a pool of blood serum from healthy patients was added in the negative selection phase ("Phase I" of FRELEX), where we select for aptamers that exhibit the capacity to bind to the immobilization field. The inclusion of healthy blood serum enables the selection against aptamers that bind to targets that are present within this material. This creates a contrast in which we are effectively selecting aptamers that bind to epitopes that are enriched in the late human AD blood pool. This selection process was repeated for a total of 10 selection rounds. Aliquots from selection rounds #5 to #10 were analyzed by NGS analysis by the Hospital for Sick Children (Toronto, Canada) using an Illumina HiSeq instrument. Sequence analysis was performed using NeoVentures Biotechnology Inc.'s proprietary software.

The aptamer library remaining from the tenth selection round ("selected aptamer library") was amplified and converted in cDNA as described before. 200 ng of cDNA was applied to blood serum from each individual subject for a single "diagnostic selection" round in a manner identical to the "Phase II" of FREELEX (*i.e.*, the positive selection step of the libraries against the late AD stage pools).

Following selection, each selected library was analyzed by next generation sequencing with an Illumina HiSeq instrument at the Hospital for Sick Children in Toronto, Canada.

*Results*

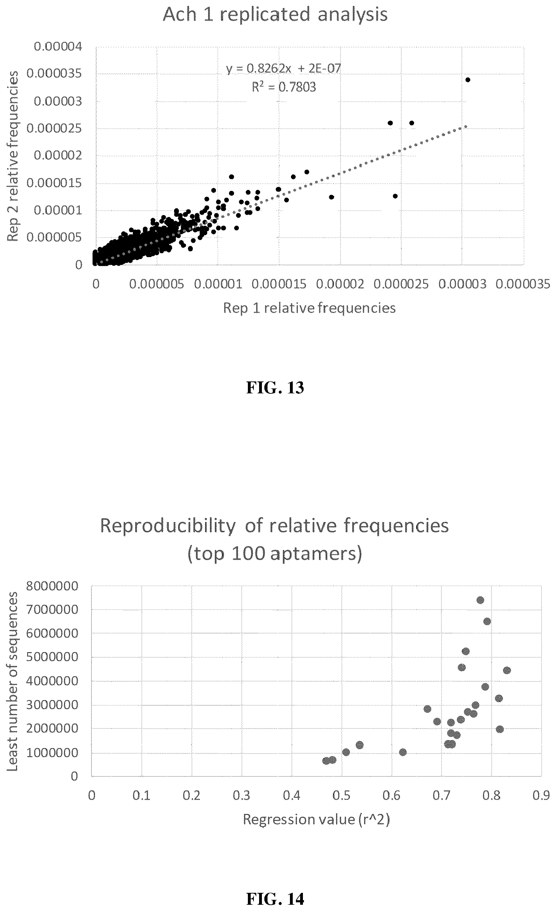

In general, the reproducibility of the relative frequency of the aptamers from one replicated analysis to the other exhibited regression values that were highly significant. In **Figure 13,** the relative frequencies of 10,000 aptamers between the two replicated analyses for the same subject (Ach 1) are provided as an example.

The number of sequences captured by each NGS process had an effect on the reproducibility of the relative frequency of the aptamers from these samples. **Figure 14** provides a comparison of the lowest number of sequences captured in either replication for each sample *versus* the regression coefficient obtained for the samples across replicates in terms of relative frequency of the 100 most highly enriched aptamers in selection round #10 (*i.e.,* the reproducibility of the relative frequencies of the top 100 aptamers).

The Inventors surmised that there was a curvilinear relationship between the number of sequences captured and the reproducibility of relative frequency of the aptamers. More than 1 million sequences needed to be analyzed in order to ensure a consistent level of reproducibility. Based on this insight, the Inventors eliminated six samples from further analysis: two healthy samples (Ach-20 and 24) and three AD samples (Ach-4, 16 and 44).

The relative frequency of each of the aptamers can be plotted in a three-dimensional graph for visual comparison (not shown). However, we needed to develop a means of quantifying the patterns seen on these three-dimensional graphs into a score. To do so, we focused on identifying those aptamers in the enriched libraries that provide the most information.

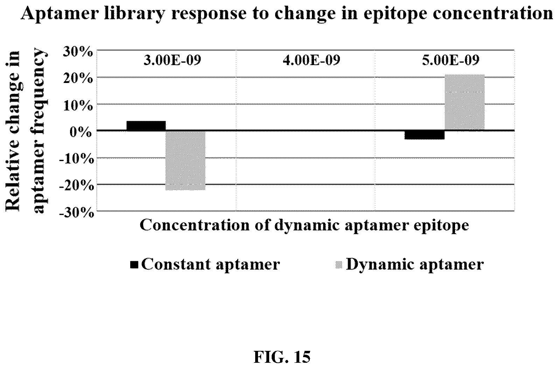

Aptamers in the library can be classified into two groups: dynamic, in that the epitope that they bind changes in concentration between healthy and AD blood samples, and constant, in that the epitope that they bind to does not change in concentration between healthy and AD blood samples. **Figure 15** illustrates this point schematically.

The constant aptamer is influenced by the action of the dynamic aptamer. If the epitope for the dynamic aptamer is at a higher concentration in a given blood sample, the constant aptamer will exhibit a decrease. The same is true vice-versa.

Our database of aptamer sequences will contain both types of aptamers, and both types will be exhibiting changes in relative frequency. We therefore wanted to identify dynamic aptamer pairs, where in a given blood sample, one member of the pair is increasing in relative frequency and the other one is decreasing.

While the relative frequency of the individual aptamers was reproducible, slight changes from one replication to another could significantly affect the aptamers that were assigned to different clusters. As a result, we developed an improvement of the invention by identifying dynamic pairs of aptamers. We define a dynamic aptamer pair, as a pair of aptamers that exhibit significant differences among individuals when the relative frequency of one is divided by the relative frequency of the other. The significance is a function of one aptamer increasing in relative frequency while the other aptamer is decreasing, or vice versa.

It is possible that there is biological meaning that underlies the behavior of such dynamic pairs. An example could be that a protein folds in a certain way in a pathological state, creating an epitope that an aptamer binds to, and folds in a different way in a healthy state, such that the same aptamer does not bind to it, but a different aptamer does. In this example, the aptamer binding to the pathological epitope would increase in relative frequency and the aptamer binding to the healthy epitope would decrease in relative frequency in a coordinated manner. That is, if one increases the other always decreases.

Other examples of possible biological meaning underlying such dynamic pairs could include the presence of a metabolite adhered to a protein in a pathological state, and the absence of such a metabolite in a healthy state. These examples only serve to illustrate the concept of potential biological meaning underlying dynamic pairs and should not be considered as complete.

The concept of dynamic pairs provides a basis for more robust interpretation of changes in relative frequency of aptamers across individuals in that such dynamic pairs implicitly increase the difference measured, and do so in a coordinated manner.

It is not necessary that such dynamic pairs actually have a paired biological basis in order to serve as an effective enablement of this invention.

To identify dynamic pairs of aptamers, we first determined the proportional relative frequency of each of the top 100 aptamers in each sample to each other. This involved dividing the relative frequency of each aptamer by the relative frequency of each other aptamer in this set of 100 aptamers.

Within each replicate, the average value for each of these proportions was calculated for the AD subjects and the healthy subjects. The difference between these average values divided by the average of the standard deviation for each value within each set (AD and healthy) was determined. This can be considered as a Z statistic.

The Z statistics from each of the replications for each individual proportion were multiplied. Z statistics that were in the same direction (negative or positive) would thus result in positive values, and Z statistics in opposite directions would result in negative values.

We then used a cut-off value of 1.2 to identify the products of the Z statistics from each replication corresponding to those aptamer pairs with the most power to differentiate AD from healthy. This resulted in the identification of 72 aptamer pairs described in **Table 3.**

**Table 3:** list of 72 dynamic aptamer pairs identified. The numbers in each pair refer to the SEQ ID NOs of Aptamarker sequences described in **Table 4.**

| Dynamic pairs | | | | | |
|---|---|---|---|---|---|
| 3/5 | 8/13 | 29/13 | 10/16 | 45/23 | 8/32 |
| 4/5 | 10/13 | 30/13 | 12/16 | 45/24 | 8/38 |
| 12/5 | 11/13 | 31/13 | 20/16 | 6/27 | 6/42 |

(continued)

| Dynamic pairs | | | | | |
|---|---|---|---|---|---|
| 45/5 | 12/13 | 34/13 | 22/16 | 8/27 | 8/42 |
| 46/5 | 14/13 | 35/13 | 28/16 | 12/27 | 21/42 |
| 45/2 | 18/13 | 36/13 | 28/17 | 20/27 | 28/42 |
| 45/9 | 19/13 | 40/13 | 35/16 | 28/27 | 45/37 |
| 2/13 | 20/13 | 41/13 | 43/16 | 30/27 | 45/38 |
| 3/13 | 22/13 | 43/13 | 45/15 | 35/27 | 45/39 |
| 4/13 | 25/13 | 45/13 | 45/16 | 45/27 | 45/42 |
| 6/13 | 26/13 | 46/13 | 45/17 | 45/32 | 45/44 |
| 7/13 | 28/13 | 8/16 | 46/16 | 45/33 | 46/39 |

Table 4: list of 45 Aptamarker sequences. The sequences correspond to the 40-mer random region (*i.e.,* $N_{40}$ part) of SEQ ID NO: 1.

| SEQ ID NOs: | Aptamarker sequences (from 5' to 3') (40-mer random region of SEQ ID NO: 1) |
|---|---|
| 2 | CCTGCATTACCGACTAGCCGCCACATAGCCACTCCTTTCA |
| 3 | GACCTCATTCCAAGCCCGCACCACAGCACCAGAGCCGATG |
| 4 | TTCGGGACCCTACGCGGCGCTCCCATCCACGCACATCCAA |
| 5 | AACCTTTTACCCTTGGCCATTACACCACTGCCATCCTGTC |
| 6 | CCCCGGTATCCCTCGAGGGCCCAGCCACAACCTGCTGCCA |
| 7 | CGCCGCCAATCATAGCAACCCGGCCATCCACATCCTGCGA |
| 8 | GATTGACCAGGGGACCCAGCCATCCGACCCCACACCAGCA |
| 9 | GCCCCTGATTCGACTCAAGGACCACCGACCATATACCTCG |
| 10 | ATTCAGGTTTGACCCCTCGACACACACACCACCATCCAGA |
| 11 | TGACCGTTATATATCCGGCTATCGACCACGACCACCTGCT |
| 12 | CTAGACAGGCGCGCTACCTATTTTTGCCACCACAGCCACG |
| 13 | TGTCCCTGCGTATCCTGAGAACCGATTACACCCACTACCA |
| 14 | CAACCTTGACTGTCGACAATTCACCGCACACCCGATCCTG |
| 15 | GAACCCACGAATTGTCAACCCGATCCAATCACCACGACCA |
| 16 | TACTGATCGATCTTCATACTCCGCCACATTGACCATCCCG |
| 17 | AGCGCCAAACAGCCATATCCCGACGATACCACATAACCGA |
| 18 | CGCCGACGGTTGCTCTCGACTTGCCCATCCCCTGCCATGA |
| 19 | GCCAGTAGACGCTGACCGCCAACCCACCGCCATCCATGAG |
| 20 | TGGCACTGGGGCTGGCCGAACACCACCCACCCTATAGCAA |
| 21 | CGAGAGACAGATATCCCTTCGCATCCACTACCAATCGCCG |
| 22 | TAGGAGCAGTACCCCGATCGACCACACCACTCTACGGGCA |
| 23 | GCCCTTGAATCGATCCCGGACTTGCCACATCCACAGCAGA |
| 24 | TGCCAACAGATGACTACTGCGCACCCATCAGCCACATCCA |
| 25 | ATCAGAACCTTACCGGCCGTCACTTTGCCACCACAACCGA |
| 26 | GAACCGTCGTCTACCTCGATCCCGCCCACAGCACTACCAG |

(continued)

| SEQ ID NOs: | Aptamarker sequences (from 5' to 3') (40-mer random region of SEQ ID NO: 1) |
|---|---|
| 27 | CACTATTTCTTACAACACGCGCGCTTTACCACCATACCGA |
| 28 | TCTGGGCCCAACAGACCTCGACCCGCTGACACCGCTACCA |
| 29 | CGCCGGCATATCGCTCTAACCCGGGACCACACCCACGACT |
| 30 | TAGCTCGGATTGAGACGCCCACCTCCACAGCCCTTGACCG |
| 31 | TAGGCCATCCCTTCATCCACGTAGCCGCCATTGACCCCGA |
| 32 | TGGCCATATGTTCTCCCATAAGCTGCACCACACCCCTTGA |
| 33 | AAGGTCGTCGAATAACCCCTCATCCACCGCCGTACCACAA |
| 34 | AGGCCGTCCTCGAACATACCTAACCACACCCCACTGACCG |
| 35 | CAGACGCCTTAACCTCTACCCGAGCCCTGCCACGCCATGT |
| 36 | GCCCCCTGCTATTACACATCGAGCATTCGCCCACACCCAG |
| 37 | CCCAGATAACCCATCCCGACCTGAGAGCCCCGATACCATA |
| 38 | CGGCTCTACCTGCTCTGTCCGTTCCACATAACCCAAACCA |
| 39 | GTGTGCTACCAACAGATAATGACCACTCCCAATGAGCGCG |
| 40 | ACCTCTATTCCCGGCGCCCCAACCACATATCCAGGACTTG |
| 41 | ATCGACTTCCACTCTAGTCTCGACCGCCCACCGTACCATA |
| 42 | CAGCCTTTTGTTAACCTGCACCCACAGTACCCGATAGACA |
| 43 | ACGGACACGCCGGCTAGCCACCCTTAACCTATATGTCG |
| 44 | TGACGCTTTAATCCGCCCCATAGCACCACACCACCAATCA |
| 45 | CAGAAACCGCTAAGCCCTTGCCACAGACCAGCATGTGCCG |
| 46 | AGCCGGAATGAGAACCCCCGACCAACCCATCCATACCGAA |

5'AACTACATGGTATGTGGTGAACT($N_{40}$)GACGTACAATGTACCCTATAGTG-3' (SEQ ID NO: 1), where N can be A, T, C or G.

All 72 of these aptamer pairs exhibited lower values for the AD average compared to the healthy average. This would not be expected as a necessary result of this analytical process as positive Z value products could be derived from proportions where the AD average was higher than the healthy average in both replicates, and where the AD average was lower than the healthy average in both replicates. Thus, a randomization set of data would be expected to yield on average half of the proportions where one average was higher than the other, and half of the proportions where one average was lower than other.

This highly significant difference from random probability distribution can only be taken as an indication of the biological meaning of the relative frequencies. This meaning is a result both of the selection process and the nature of the differences in the relative frequency of epitopes in the blood of AD and healthy subjects.

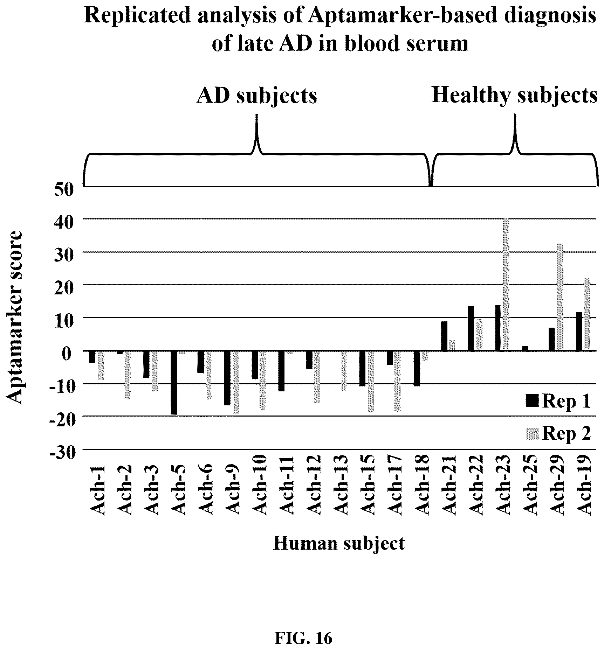

Given that all of the values were in the same direction, we summed the proportions of relative frequencies for the same 72 aptamer pairs within each individual sample. These values are provided in **Figure 16.** To create an ordinate of zero between the AD values and the healthy values, a constant operator value of 2.7 was subtracted from all sums. This final value is referred to as an "Aptamarker score".

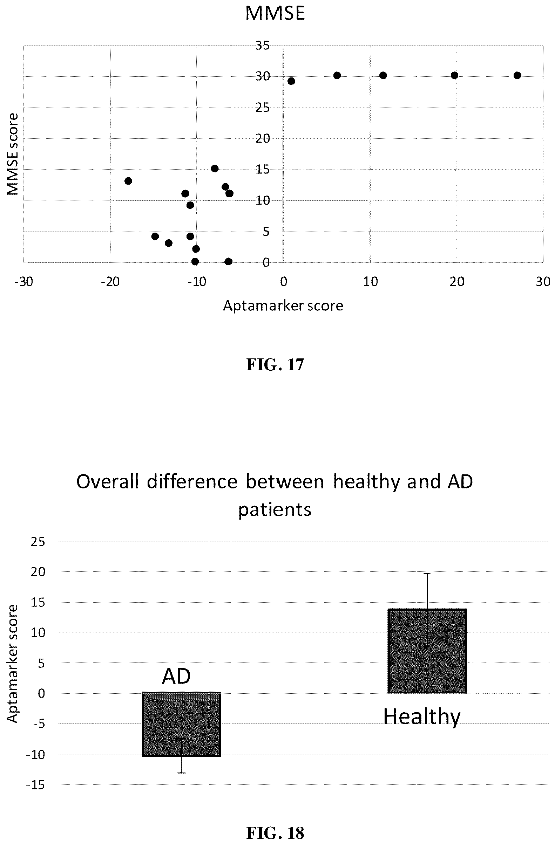

A comparison of the average "Aptamarker score" value for each subject to their MMSE score is provided in **Figure 17.** It is clear that blood analysis of human subjects with the Aptamarker platform can be effectively and reliably used to differentiate subjects on the basis of a reduction in MMSE score.

**Figure 18** provides an overall summary of the differentiation between AD subjects and healthy subjects based on this analysis.

*Conclusion*

It is clear from these data that we can use the Aptamarker platform to distinguish between patients affected by Alzheimer's disease and healthy patients based on their blood sample.

Correlation between the Aptamarker fingerprint and the degree of disease severity will be analyzed by training aptamer libraries on mid-stage Alzheimer's disease samples.

**Example 4: Randomized analysis of late AIBL data**

In our study described in Example 3, we had 13 AD and 6 healthy subjects. A random grouping was set up with the same distribution, but four of the healthy subjects were randomly assigned to the AD group, and four AD subjects were randomly assigned to the healthy group.

Dynamic aptamer pairs were chosen in the same manner as for the original analysis of Example 3. 46 such pairs were identified.

The first difference observed was that the difference between the average for the AD subjects *versus* the healthy subjects was not all in the same direction. In the analysis provided in Example 3, all 72 dynamic aptamer pairs exhibited higher values in the healthy pool than in the AD pool. In this randomized analysis, in the first replication, 30 of the dynamic pairs exhibited higher values in the AD pool. In the second replication, 17 of the same dynamic pairs exhibited higher values in the AD pool.

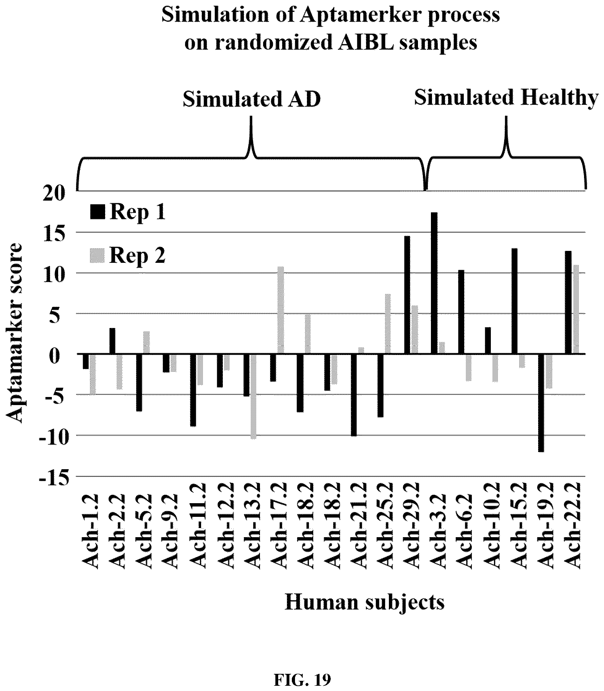

The best course of action was therefore to add those values that had lower values in the AD pool than in the healthy pool, and subtract those that had higher values in the AD pool than in the healthy pool. This algorithm was established for the first replication. **Figure 19** summarizes the results across both replications.

The algorithm was fitted to the first replication and the results were not as good as in the analysis of Example 3 (**Figure 16**). The results obtained by fitting this algorithm to the data from the second replication show no particular trend at all. In the analysis of Example 3 though, the same algorithm was used for both replicated sets of data.

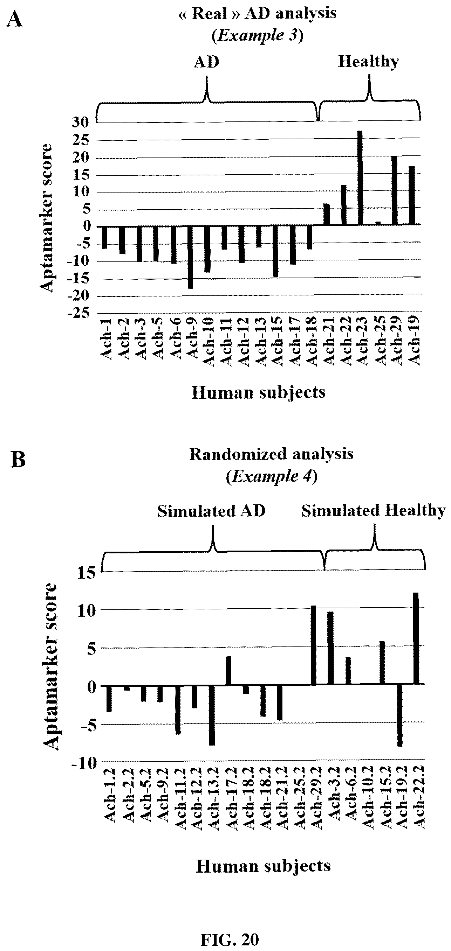

**Figure 20** provides a comparison between the overall averages per human subject in the analysis of Example 3 **(Figure 20A)** and the simulated randomized analysis of the present Example **(Figure 20B).**

These results clearly demonstrate that the differentiation achieved between AD and healthy subjects with Aptamarkers is not a function of the statistical process used to identify the dynamic pairs of aptamers, but reflects biological reality.

**Conclusion**

The rate of progression of Alzheimer's disease can vary greatly across patients. There is a need to project this rate on an individual basis as an aide to health care planning. It would also be useful to be able to characterize the rate of pathology development as a means of characterizing the performance of candidate therapies.

We have contemplated, using repeated analysis of blood serum from patients affected by Alzheimer's disease, to characterize the rate at which the disease is progressing within each patient. Differences in the rates of AptaMarker enrichment will be correlated to the rate of disease progression and used to project rates of decline on a diagnostic basis.

It has also not escaped our attention that this AptaMarker platform has the capacity to identify subgroups of patients, or subtypes of the pathology on the basis of differences in epitopes, or differences in the rate of accumulation of epitopes. As such, the application of this platform would be very useful as a guide for the stratification of patients for clinical trials of candidate therapeutics. In addition, the capacity of this approach to characterize the progression of epitopes indicative of the disease would make the use of this approach attractive as a companion diagnostic for successful therapies.

It has also not escaped our attention that an improvement to this process could involve the use of a subset of the aptamers, for instance but not limited, to the aptamers involved in the 72 dynamic pairs identified in Example 3. Such a set could be synthesized in order to ensure that aptamer distribution is the same in each test applied. The use of such a subset would also result in an increase in the magnitude of individual aptamer response to changes in epitope frequency among subjects analyzed.

**Claims**

1. A method for diagnosing a medical state, disease or condition in a subject, comprising the steps of:

- a) providing a selected aptamer library, wherein the selected aptamer library comprises a collection of aptamer sequences selected and optionally counter-selected against a bodily tissue or fluid from at least one reference subjects,
- b) contacting the selected aptamer library with a biological sample from at least one subject with known outcome for the medical state, disease or condition,

- c) selecting aptamers from the selected aptamer library that bind to the biological sample, wherein the selected aptamer library comprises from 1 to $10^{11}$ oligonucleotide sequences, thereby obtaining a subject-specific aptamer library,

- d) determining the relative frequency of each aptamer of each of the at least one reference subject-specific aptamer libraries obtained in the previous step, then determining the changes of these relative frequencies between the selected aptamer library of step a) and each reference subject-specific aptamer library of step c),

- e) correlating changes in relative frequency between aptamers from the selected aptamer library and the subject-specific aptamer library to the medical state, disease or condition of the at least one subject, and

- f) applying the selected aptamer library to subjects for which the medical state, disease or condition is unknown, thereby diagnosing the subject for the medical state, disease or condition based on the correlations determined in the previous step between the relative change of aptamers within the library and said medical state, disease or condition,

wherein the at least one reference subject is a subject with known outcome for the medical state, disease or condition.

2. The methods according to claim **1,** wherein the medical state, disease or condition is a neurodegenerative disease.

3. The methods according to claim **1** or claim **2,** wherein the medical state, disease or condition is Alzheimer's disease.

4. The method according to any one of claims **1** to **3,** wherein step a) comprises counter-selecting aptamer sequences against a bodily tissue or fluid from at least one other reference subject who is/was not diagnosed with the medical state, disease or condition, or against a pool of bodily tissues or fluids from several other reference subjects who are/were not diagnosed with the medical state, disease or condition.

5. The method according to any one of claims **1** to **4,** wherein step e) comprises training an algorithm to correlate the changes in relative frequency with the medical state, disease or condition of the at least one reference subject

6. The method according to any one of claims **1** to **5,** wherein the step of contacting the selected aptamer library with a biological sample comprises two or more selection rounds.

7. The method according to any one of claims **1** to **6,** wherein the selected aptamer library comprises a subset of known and characterized aptamer sequences at known ratios.

8. The method according to any one of claims **1** to **7,** wherein the change in relative frequency is determined at various time points, thereby assessing the rate of progression of the medical state, disease or condition in the subject.

9. The method according to any one of claims **1** to **8,** wherein the method is for assigning a probability of the medical state, disease or condition to occur in the subject.

10. The method according to any one of claims **1** to **8,** wherein the method is for diagnosing the level of severity of the medical state, disease or condition in the subject.

11. The method according to any one of claims **1** to **8,** wherein the method is for determining the rate of progression of the medical state, disease or condition in the subject.

12. The method according to any one of claims **1** to **8,** wherein the method is for stratifying the subject affected with the medical state, disease or condition.

13. The method according to any one of claims **1** to **8,** wherein the method is for evaluating the efficacy of a therapy in the subject affected with the medical state, disease or condition.

**FIG. 1**

**FIG. 2**

**FIG. 3**

**FIG. 4**

**FIG. 5**

**FIG. 6**

**FIG. 7**

**FIG. 8**

**FIG. 9**

**FIG. 10**

**FIG. 11**

FIG. 12

## Ach 1 replicated analysis

$y = 0.8262x + 2E-07$
$R^2 = 0.7803$

**FIG. 13**

## Reproducibility of relative frequencies (top 100 aptamers)

**FIG. 14**

Aptamer library response to change in epitope concentration

FIG. 15

FIG. 16

MMSE

FIG. 17

Overall difference between healthy and AD patients

FIG. 18

**Simulation of Aptamerker process
on randomized AIBL samples**

FIG. 19

FIG. 20

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2017035666 A

- US 20150087536 A

**Non-patent literature cited in the description**

- **STRIMBU ; TAVEL.** *Curr Opin HIV AIDS,* November 2010, vol. 5 (6), 463-466
- **BEREZOVSKI et al.** *J Am Chem Soc.,* 16 July 2008, vol. 130 (28), 9137-43
- **ELLIS et al.** *Int Psychogeriatr,* August 2009, vol. 21 (4), 672-87
- **TIERNEY et al.** *Neurology,* March 1988, vol. 38 (3), 359-64
- **ALBERT et al.** *Alzheimers Dement,* May 2011, vol. 7 (3), 270-9
- **FOLSTEIN et al.** *J Psychiatr Res.,* November 1975, vol. 12 (3), 189-98